# EUROPEAN PATENT APPLICATION

(11) **EP 1 209 234 A1**
(43) Date of publication of application: **29.05.2002**
(21) Application number: 00956828.8
(22) Date of filing: 30.08.2000
(51) Int. Cl.: C12N 15/60, C12N 9/88, G06F 17/30

(54) **STEREOSTRUCTURE OF DECARBAMYLASE AND METHOD OF USING THE SAME**

(30) Priority: 31.08.1999 JP 24679799
(71) Applicant: Kaneka Corporation, Osaka-shi, Osaka 530-0005 (JP)
(72) Inventor: NAKAI, Takahisa, Kobe-shi, Hyogo 655-0853 (JP); MORIKAWA, Souichi, Himeji-shi, Hyogo 670-0001 (JP); ISHII, Kiyoto, Akashi-shi, Hyogo 673-0869 (JP); NANBA, Hirokazu, Takasago-shi, Hyogo 676-0025 (JP); YAJIMA, Kazuyoshi, Akashi-shi, Hyogo 673-0005 (JP); IKENAKA, Yasuhiro, Kobe-shi, Hyogo 651-2242 (JP); TAKAHASHI, Satomi, Kobe-shi, Hyogo 655-0851 (JP)
(74) Representative: Nevant, Marc
(86) International application number: JP0005901
(87) International publication number: WO0116337

(57) **Abstract**

The present invention reveals the stereostructure of decarbamylase by X-ray crystallography and provides a decarbamylase mutant, which is more useful for industrial applications, obtained by molecular design utilizing the stereostructure for the purpose of improving the reactivity of decarbamylase to D-N-carbamoyl-α-amino acid which is a substrate thereof. Specifically, the present invention relates to the stereostructure of decarbamylase determined by X-ray crystallography; a stereostructure model of a decarbamylase mutant; a stereostructure model of a complex thereof with a substrate, a product, and the like; a molecular design method utilizing these stereostructures; a decarbamylase mutant obtained by the method; and a method for designing and producing a protein mutant having a structure similar to that of decarbamylase.

SEQUENCE LISTING

<110> KANEKA CORPORATION

<120> Stereostructure of decarbamylase and method of using
      the same

<130> H23460-94EP

<140> 00956828.8
<141> 2000-08-30

<150> PCT/JP00/05901
<151> 2000-08-30

<150> JP11-246797
<151> 1999-08-31

<160> 2

<170> PatentIn Ver. 2.1

<210> 1
<211> 303
<212> PRT
<213> Agrobacterium sp.

<400> 1
Thr Arg Gln Met Ile Leu Ala Val Gly Gln Gln Gly Pro Ile Ala Arg
  1               5                  10                  15

Ala Glu Thr Arg Glu Gln Val Val Val Arg Leu Leu Asp Met Leu Thr
             20                  25                  30

Lys Ala Ala Ser Arg Gly Ala Asn Phe Ile Val Phe Pro Glu Leu Ala
         35                  40                  45

Leu Thr Thr Phe Phe Pro Arg Trp His Phe Thr Asp Glu Ala Glu Leu
     50                  55                  60

Asp Ser Phe Tyr Glu Thr Glu Met Pro Gly Pro Val Val Arg Pro Leu
 65                  70                  75                  80

Phe Glu Lys Ala Ala Glu Leu Gly Ile Gly Phe Asn Leu Gly Tyr Ala
                 85                  90                  95

Glu Leu Val Val Glu Gly Gly Val Lys Arg Arg Phe Asn Thr Ser Ile
            100                 105                 110

Leu Val Asp Lys Ser Gly Lys Ile Val Gly Lys Tyr Arg Lys Ile His
        115                 120                 125

Leu Pro Gly His Lys Glu Tyr Glu Ala Tyr Arg Pro Phe Gln His Leu
    130                 135                 140

Glu Lys Arg Tyr Phe Glu Pro Gly Asp Leu Gly Phe Pro Val Tyr Asp
145                 150                 155                 160

Val Asp Ala Ala Lys Met Gly Met Phe Ile Cys Asn Asp Arg Arg Trp
                165                 170                 175

Pro Glu Ala Trp Arg Val Met Gly Leu Arg Gly Ala Glu Ile Ile Cys
            180                 185                 190

Gly Gly Tyr Asn Thr Pro Thr His Asn Pro Pro Val Pro Gln His Asp
        195                 200                 205

His Leu Thr Ser Phe His His Leu Leu Ser Met Gln Ala Gly Ser Tyr
    210                 215                 220

Gln Asn Gly Ala Trp Ser Ala Ala Ala Gly Lys Val Gly Met Glu Glu
225                 230                 235                 240

Asn Cys Met Leu Leu Gly His Ser Cys Ile Val Ala Pro Thr Gly Glu
                245                 250                 255

Ile Val Ala Leu Thr Thr Thr Leu Glu Asp Glu Val Ile Thr Ala Ala
            260                 265                 270

Val Asp Leu Asp Arg Cys Arg Glu Leu Arg Glu His Ile Phe Asn Phe
        275                 280                 285

Lys Gln His Arg Gln Pro Gln His Tyr Gly Leu Ile Ala Glu Leu
    290                 295                 300

<210> 2
<211> 303
<212> PRT
<213> Escherichia coli

<400> 2
Thr Arg Gln Met Ile Leu Ala Val Gly Gln Gln Gly Pro Ile Ala Arg
1               5                   10                  15

Ala Glu Thr Arg Glu Gln Val Val Val Arg Leu Leu Asp Met Leu Thr
            20                  25                  30

Lys Ala Ala Ser Arg Gly Ala Asn Phe Ile Val Phe Pro Glu Leu Ala
        35                  40                  45

Leu Thr Thr Phe Phe Pro Arg Trp Tyr Phe Thr Asp Glu Ala Glu Leu
    50                  55                  60

Asp Ser Phe Tyr Glu Thr Glu Met Pro Gly Pro Val Val Arg Pro Leu
65                  70                  75                  80

Phe Glu Lys Ala Ala Glu Leu Gly Ile Gly Phe Asn Leu Gly Tyr Ala
                85                  90                  95

Glu Leu Val Val Glu Gly Gly Val Lys Arg Arg Phe Asn Thr Ser Ile
            100                 105                 110

Leu Val Asp Lys Ser Gly Lys Ile Val Gly Lys Tyr Arg Lys Ile His
        115                 120                 125

Leu Pro Gly His Lys Glu Tyr Glu Ala Tyr Arg Pro Phe Gln His Leu
    130                 135                 140

Glu Lys Arg Tyr Phe Glu Pro Gly Asp Leu Gly Phe Pro Val Tyr Asp
145                 150                 155                 160

Val Asp Ala Ala Lys Met Gly Met Phe Ile Cys Asn Asp Arg Arg Trp
                165                 170                 175

Pro Glu Ala Trp Arg Val Met Gly Leu Arg Gly Ala Glu Ile Ile Cys
            180                 185                 190

Gly Gly Tyr Asn Thr Pro Thr His Asn Pro Glu Val Pro Gln His Asp
        195                 200                 205

His Leu Thr Ser Phe His His Leu Leu Ser Met Gln Ala Gly Ser Tyr
    210                 215                 220

Gln Asn Gly Ala Trp Ser Ala Ala Ala Gly Lys Ala Gly Met Glu Glu
225                 230                 235                 240

Asn Cys Met Leu Leu Gly His Ser Cys Ile Val Ala Pro Thr Gly Glu
                245                 250                 255

Ile Val Ala Leu Thr Thr Thr Leu Glu Asp Glu Val Ile Thr Ala Ala
            260                 265                 270

Val Asp Leu Asp Arg Cys Arg Glu Leu Arg Glu His Ile Phe Asn Phe
        275                 280                 285

Lys Gln His Arg Gln Pro Gln His Tyr Gly Leu Ile Ala Glu Leu
    290                 295                 300

## Description

The present invention relates to a crystal of an enzyme which converts D-N-carbamoyl-α-amino acids to corresponding D-α-amino acids (hereinafter referred to as decarbamylase). The present invention also relates to the stereostructure of decarbamylase determined by X-ray crystallography using the crystal, and the use thereof, and more particularly, to methods for designing amino acid mutations to improve the stability of the enzyme (e.g., heat resistance, organic solvent resistance, air oxidation resistance, etc.), change the optimum pH of the enzyme reaction, and improve the specific activity of decarbamylase by utilizing its stereostructure. Moreover, the present invention relates to a method for producing decarbamylase mutants by utilizing the above-described stereostructure, resultant decarbamylase mutants, and uses thereof.

### BACKGROUND ART

Optically active D-α-amino acids are valuable compounds as pharmaceutical intermediates. Particularly, examples of the D-α-amino acids, which are industrially useful, include D-phenylglycine and D-para-hydroxyphenylglycine which are intermediates in the production of semi-synthetic penicillins or semi-synthetic cephalosporins. As a method for producing such D-α-amino acids, a method comprising removing a carbamoyl group from a corresponding D-N-carbamoyl-α-amino acid so as to obtain the D-α-amino acids is known. In this case, the removal of a carbamoyl group is conducted by a chemical method or a method utilizing an enzyme reaction of a microorganism.

An enzyme which removes a carbamoyl group is called decarbamylase. This enzyme catalyzes the conversion of D-N-carbamoyl-α-amino acid to D-α-amino acid. This enzyme has been identified from bacteria of the genera *Pseudomonas, Agrobacterium, Aerobacter, Aeromonas, Brevibacterium, Bacillus, Flavobacterium, Serratia, Micrococcus, Arthrobacter, Alcaligenes, Achromobacter, Moraxella, Paracoccus, Blastbacter,* and *Comamonas.* The amino acid sequence and/or nucleic acid sequence of decarbamylase has been determined from the genus *Agrobacterium* (e.g., *Agrobacterium radiobacter* NRRL B11291 and *Agrobacterium* sp. KNK712 (Kaneka bacterium)).

In general, most enzymes are not stable to the point of being resistant to conditions under which the enzymes are industrially used in reactions at room temperature or high temperature, and the stability of enzymes often has an influence on the cost of products. A so-called "bioreactor", such as immobilized enzyme or immobilized bacterium, is used to repeat reactions in order to cause an enzyme reaction to proceed advantageously. In this case, there is also a limit to the number of times an enzyme can be used due to the stability of the enzyme, exerting an influence on the cost of products.

For the purpose of effectively using an enzyme, which catalyzes an useful reaction, in industrial applications, there is a demand for a method for rapidly or efficiently creating an enzyme having excellent physical properties (e.g., stability) or a high catalytic activity. As a method of obtaining an enzyme having an improved physical property or function, a so-called random screening method is known, in which a gene encoding an enzyme is artificially mutated by chemical treatment, enzyme treatment, or the like, the recombinant DNA having the mutation is introduced into a host cell, and an enzyme having an intended function and/or physical property is screened. On the other hand, with the recent advances in structural biology, the stereostructure of a number of proteins including enzymes have been revealed by X-ray crystallography or NMR analysis and, using stereostructure and molecular design techniques, modification of the physical properties or functions of proteins has been increasingly conducted.

The term "the stereostructure of a protein" as used herein refers to the three-dimensional structure of the protein defined under certain conditions and by the amino acid sequence of the protein, i.e., the protein having the amino acid sequence is folded into the three-dimensional structure under the certain conditions. The stereostructure of a protein may be determined by X-ray crystallography or nuclear magnetic resonance.

The modification of the physical properties of decarbamylase with the random screening method has been successfully carried out to obtain an enzyme-producing bacterial strain (E. coli JM109) having improved heat resistance, which is an industrially useful decarbamylase mutant (International Publication WO94/03613). Further, a mutant having improved stability has been obtained by site-specific mutagenesis (Japanese Laid-Open Publication No. 9-173068). These examples are mutants which were produced based on the primary amino acid sequences thereof, but not by means of a rational molecular design method utilizing the stereostructure of enzymes.

The stereostructure of decarbamylase and similar enzymes has not been determined. It has been found from sequence similarity analysis for proteins, whose amino acid sequences had already been known, using a protein sequence database, PIR Release57 (National Center for Biotechnology Information (NCBI)) that decarbamylase has a weak sequence similarity of about 25-30% to hydrolase, such as amidase and nitrilase. However, none of the stereostructures of these enzymes having weak sequence similarity have been determined. Therefore, it is practically impossible to estimate the stereostructure of decarbamylase by a so-called homology modeling technique using the stereostructures of similar proteins, which is often used for the molecular design technique (e.g., Swiss-Pdbviewer (modeling program) (Swiss Institute of Bioinformatics (SIB), ExPASy Molecular Biology Server (available from http://www.expasy.ch/)); Guex, N. and Peitsch, M.C. (1997) SWISS-MODEL and the Swiss-PdbViewer: An environment for comparative protein modeling, Electrophoresis 18, 2714-2723). Even if the stereostructure of a similar enzyme is determined, it is difficult to obtain, based on a sequence similarity of less than 30%, the stereostructure model of decarbamylase with a sufficient accuracy for application of a reasonable molecular design method. The accurate atomic coordinate data of decarbamylase is required to predict amino acid mutations involved in a change in the optimum pH of an enzyme reaction, an improvement in specific activity, and the like with high precision for the purpose of molecular design. If the stereostructure of decarbamylase can be determined, precise analysis of the stereostructure and a reasonable molecular design technique based thereon can be conducted, thereby further making it possible to rapidly or efficiently obtain a modified enzyme which is advantageous in industrial applications.

### (Problem to be Solved by the Invention)

To solve the above-described problems, an object of the present invention is to obtain a single crystal of decarbamylase which is currently used in industrial appliations, and reveal the stereostructure of decarbamylase with X-ray crystallography. Another object of the present invention is to provide a decarbamylase mutant which is more advantageous in industrial applications by conducting molecular design for the purpose of an improvement of the reactivity with D-N-carbamoyl-α-amino acids (i.e., the substrate of decarbamylase), optimization of reaction pH, the stability against heat and air oxidation, and a method for producing D-α-amino acids using the obtained decarbamylase mutant.

### DISCLOSURE OF THE INVENTION

### (Means for Solving the Problem)

The inventors of the present invention have studied vigorously to solve the above-described problem. As a result, a single crystal of decarbamylase and a heavy atom derivative crystal thereof were obtained. The precise stereostructure of decarbamylase was determined by X-ray crystallography using a heavy atom isomorphous replacement method and a multi-wavelength anomalous dispersion method. Based on the stereostructure, a mutant having improved characteristics were produced. The present invention was thus completed.

The present invention relates to decarbamylase crystal having a space group P2₁2₁2 in the orthorhombic system and an amino acid sequence set forth in SEQ ID NO.: 1, or a space group P2₁2₁2₁ in the orthorhombic system and an amino acid sequence set forth in SEQ ID NO.: 2. The present invention relates to decarbamylase crystal having a space group P2₁2₁2 and an amino acid sequence set forth in SEQ ID NO.: 1, or a space group P2₁2₁2₁ in the orthorhombic system and an amino acid sequence set forth in SEQ ID NO.: 2. In one embodiment, the crystal may have a unit cell in the form of a rectangular parallelepiped and has lattice constants: a=66.5-68.5 Å, b=135.5-138.0 Å, and c=66.5-68.5 Å. The amino acid sequence may be SEQ ID NO.: 1. In another embodiment, the crystal may have a unit cell in the form of a rectangular parallelepiped and has lattice constants: a=68.5-70.5 Å, b=138.0-140.5 Å, and c=68.5-73.0 Å. The amino acid sequence may be SEQ ID NO.: 1. In another embodiment, the crystal may have a unit cell in the form of a rectangular parallelepiped and has lattice constants: a=81.5-82.5 Å, b=133.0-135.0 Å, and c=119.5-121.5 Å. The amino acid sequence may be SEQ ID NO.: 2. In one aspect, the present invention may provide a crystal which contains at least one or more heavy metal atoms per decarbamylase molecule. In one embodiment, the heavy metal atom may be any of mercury, gold, platinum, lead, iridium, osmium, and uranium. In another embodiment, the present invention may provide frozen crystal, prepared by freezing decarbamylase crystal in liquid nitrogen.

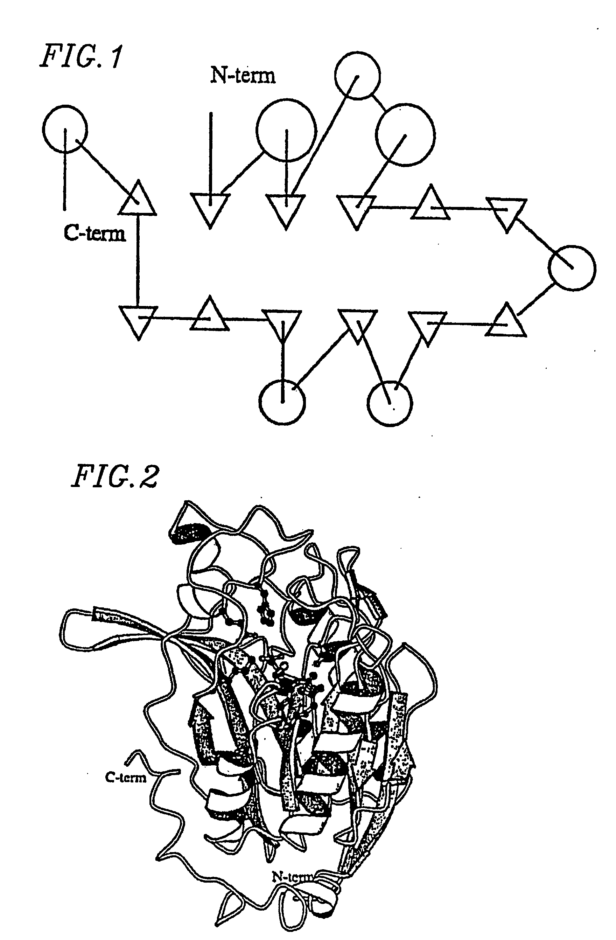

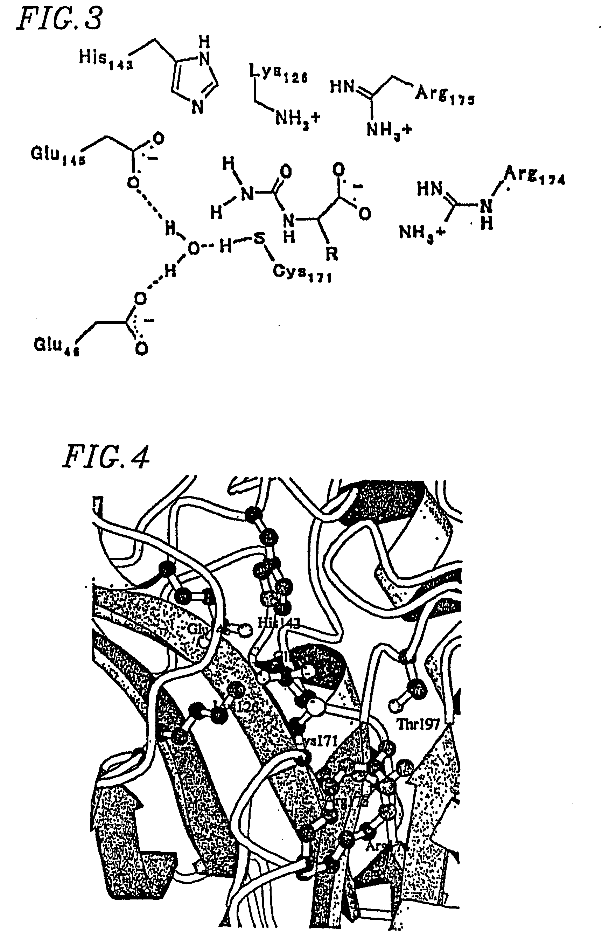

In another aspect, the present invention provides a method for preparing a crystal of decarbamylase, comprising the steps of: providing decarbamylase solution having a concentration of 1-50 mg/ml; providing precipitant solution containing polyethylene glycol (PEG) or methoxypolyethylene glycol (PEGMME) having a concentration of 5-30 wt%, and a buffer agent having a concentration such that pH 6.0-9.0 is provided; mixing the decarbamylase solution with the precipitant solution; and allowing the resultant mixture solution to stand for a predetermined period of time until the decarbamylase crystal is grown in the solution to a predetermined size or more. In one embodiment, in the method of present invention, the mixing step comprises mixing a droplet of the decarbamylase solution with a droplet of the precipitant solution, and the step of allowing the resultant mixture solution to stand comprises suspending the mixture droplet obtained in the mixing step on a solution reservoir holding the precipitant solution in a sealed container. The precipitant solution in the solution reservoir has a vapor pressure lower than a vapor pressure of the mixture droplet. Also, in the method of present invention, the mixing step comprises mixing a droplet of the decarbamylase solution with a droplet of the precipitant solution, and the step of allowing the resultant mixture solution to stand comprises suspending the mixture droplet obtained in the mixing step on a droplet stage of a solution reservoir holding the precipitant solution in a sealed container. The precipitant solution in the solution reservoir has a vapor pressure lower than a vapor pressure of the mixture droplet. In another embodiment, the predetermined period of time during which the mixture solution is allowed to stand is one day to three weeks. In another embodiment, the method further comprises placing the decarbamylase solution in a size exclusion semi-permeable membrane after the step of providing the decarbamylase solution. The mixing step comprises diffusing the precipitant solution through the semi-permeable membrane into the decarbamylase solution. In another embodiment, the mixing step comprises gradually adding the precipitant solution to the decarbamylase solution, and the step of allowing the resultant mixture solution to stand comprises allowing the mixture solution to stand in a sealed container. In another embodiment, the present invention relates to decarbamylase characterized by a stereostructure having protein stereostructure topology represented in Figure 1. In one embodiment, the present invention relates to decarbamylase having a four-layer sandwich structure containing a secondary structure containing four a helices and twelve β sheets. In a preferred embodiment of the present invention, amino acid residues involved in an enzyme reaction are one cysteine residue, two glutamic acid residues; one lysine residue, and a substrate of the enzyme reaction is D-N-carbamoyl-α-amino acid; and a substrate-binding active site thereof is characterized by a stereostructure having a substrate-binding active site represented by Figure 3. In another embodiment of the present invention, the present invention relates to an enzyme molecule having decarbamylase activity wherein a substrate thereof is D-N-carbamoyl-α-amino acid, wherein the enzyme molecule has an active site cavity formed of at least amino acids corresponding to the following amino acids of SEQ ID NO.: 1 or 2: Glu at position 46, Lys at position 126, Glu at position 145, and Cys at position 171. In another embodiment, in a reaction, the D-N-carbamoyl-α-amino acid can interact with amino acids corresponding to Lys at position 126, His at position 143, Glu at position 145, Arg at position 174, Arg at position 175, and Thr at position 197 of SEQ ID NO.: 1 or 2 at the active site cavity. In another embodiment, amino acids corresponding to Glu at position 46, Glu at position 145, and Cys at position 171 of SEQ ID NO.: 1 or 2 have a hydrogen bond via a water molecule at the active site cavity. In another embodiment, the D-N-carbamoyl-α-amino acid is selected from the group consisting of D-N-carbamoyl-phenylglycine, D-N-carbamoyl-parahydroxyphenylglycine, D-N-carbamoyl-phenylalanine, D-N-carbamoyl-valine, D-N-carbamoyl-alanine, D-N-carbamoyl-cysteine, D-N-carbamoyl-aspartic acid, D-N-carbamoyl-glutamic acid, D-N-carbamoyl-glycine, D-N-carbamoyl-histidine, D-N-carbamoyl-isoleucine, D-N-carbamoyl-lysine, D-N-carbamoyl-leucine, D-N-carbamoyl-methionine, D-N-carbamoyl-asparagine, D-N-carbamoyl-proline, D-N-carbamoyl-glutamine, D-N-carbamoyl-arginine, D-N-carbamoyl-serine, D-N-carbamoyl-threonine, D-N-carbamoyl-tryptophan, and D-N-carbamoyl-tyrosine. In another embodiment, the present invention relates to a decarbamylase complex characterized by a stereostructure of a complex of decarbamylase, a mutant thereof, or an active fragment thereof, and D-N-carbamoyl-α-amino acid or D-α-amino acid. The complex is constructed with a molecular design technique from a stereostructure of decarbamylase.

In one aspect of the present invention, the present invention relates to a method for designing decarbamylase mutants, comprising the step of designing the decarbamylase mutants having a physical property and/or a function modified based on a stereostructure of decarbamylase according to any one of claims 14, 16, and 21. In another aspect, the present invention relates to a method for designing decarbamylase mutants, comprising the step of: producing a crystal of an enzyme having decarbamylase activity; determining a stereostructure of the crystal by subjecting the crystal to X-ray crystallography; and designing the decarbamylase mutants having an improved physical property and/or function based on the determined stereostructure. In another aspect, the present invention relates to a method for designing decarbamylase mutants, comprising the step of: preparing a crystal of an enzyme having decarbamylase activity; determining a stereostructure of the crystal by subjecting the crystal to X-ray crystallography; designing the decarbamylase mutants having an improved physical property and/or function based on the determined stereostructure; and producing the decarbamylase mutants. In one embodiment, the stereostructure used in the method of the present invention is a stereostructure of decarbamylase of the present invention. In another embodiment, the step of designing the decarbamylase mutants is intended for a modification of one or more characteristics of the enzyme selected from the group consisting of a change in substrate specificity, a change in specific activity, an improvement in stability, optimization of optimum pH, and a change in water solubility. In another embodiment, the production method of decarbamylase mutants is intended to improve stability. Preferably, the mutant designing for an improvement in stability includes a mutation including substitution of an amino acid residue which leads to a reduction in activity due to air oxidation. In another embodiment, the modification of the characteristics of the enzyme includes a change in specific activity and optimization of optimum pH.

In another aspect, the present invention relates to a decarbamylase mutant obtained with the method of the present invention. The present invention also relates to a method for screening and/or designing an inhibitor for decarbamylase by utilizing the stereostructure of the present invention. In another aspect, the present invention provides a method for modifying other polypeptide or protein enzymes having an at least 30% primary amino acid sequence similarlity to decarbamylase by utilizing the stereostructure of a decarbamylase crystal or decarbamylase.

### BRIEF DESCRIPTION OF THE DRAWINGS

Figure **1** is a diagram showing the protein stereostructure topology of decarbamylase. The β strand structure is indicated by triangles. The a helix structure is indicated by circles. "N-term" indicates the terminus of a network. "C-term" indicates the carboxy terminus.
Figure **2** is a ribbon-strand diagram of decarbamylase. The β strand structure is indicated by plates. The a helix structure is indicated by helices. The amino acid side chains involved in catalytic activity are indicated by ball and stick notation.
Figure **3** is a schematic diagram showing the substrate binding manner of the active site of decarbamylase. R-indicates the side chain of a D-α-amino acid. The names of amino acids are indicated by residue numbers and triple-character notation.
Figure **4** is a diagram showing the stereostructure of the catalytic active site of decarbamylase. The β strand structure is indicated by sheets. The a helix structure is indicated by helices. The amino acid side chains involved in catalytic activity are indicated by ball and stick notation. The names of amino acids are indicated by residue numbers and triple-character notation.
Figure **5** is a diagram showing a Harker plane of a difference Patterson map calculated from 1.00 Å and 0.98 Å diffraction data.
Figure **6** is a diagram showing a Harker plane of a difference Patterson map calculated from 1.00 Å and 1.27 Å diffraction data.
Figure **7** is a diagram showing a Harker plane of an anomalous Patterson map calculated where 0.98 Å is regarded as anomalous data.
Figure **8** is a representative electron density map of the catalytic active site, indicating the stereostructure of the present invention.

### BEST MODE FOR CARRYING OUT THE INVENTION

Hereinafter, the present invention will be described in detail.

The term "decarbamylase activity" as used herein refers to the activity to convert D-N-carbamoyl-α-amino acid to D-α-amino acids by removing a carbamoyl group modifying the amino acid. The term "decarbamylase" refers to an enzyme having decarbamylase activity. Examples of decarbamylase include an enzyme having an amino acid sequence of SEQ ID NO.: 1 or SEQ ID NO.: 2. Decarbamylase of SEQ ID NO. : 1 was isolated from *Agrobacteriu*m sp. KNK712, and sequenced. The enzyme having the amino acid sequence of SEQ ID NO.: 2 is an enzyme that was obtained by screening mutated E. coli strains having randomly mutated SEQ ID NO.: 1.

The term "mutant" as used herein like "a mutant of decarbamylase or other enzymes" refers to an enzyme which has an amino acid sequence having at least one amino acid substitution, addtion, or deletion, or a modified amino acid sequence, while keeping at least part of the activity of the original enzyme. The term "active fragment" refers to a fragment (part) of the amino acid sequence of a certain protein enzyme or polypeptide enzyme, which keeps at least part of the activity of the original enzyme. In this case, "at least part of the activity" represetatively refers to at least 10% of the specific activity of the original enzyme and preferably at least 50% of the specific activity of the original enzyme, or if desired, less than 10% of the specific activity.

The term "native crystal" as used herein refers to a single crystal of decarbamylase which is grown in buffer containing an appropriate composition of a precipitant (e.g., ammonium sulfate, polyethylene glycol, etc.), an additive salt, and the like, and does not contain a heavy metal atom.

The "heavy atom derivative crystal" as used herein refers to any one of the crystals obtained in the following manners: (i) a prepared native crystal is soaked in a solution containing a heavy metal compound (e.g., mercury, gold, platinum, lead, iridium, osmium, uranium, etc.), so that heavy metal atoms are coupled with decarbamylase in the crystal via covalent bonds or coordinate bonds without disturbing the crystallinity, (ii) decarbamylase is grown to single crystal in buffer solution containing an appropriate composition of a precipitant (e.g., ammonium sulfate, polyethylene glycol, etc.), an additive salt, and the like, and also containing the above-described heavy metal compound at an appropriate concentration, and (iii) a decarbamylase mutant in which a methionine and/or cysteine residue is substituted with selenomethionine and/or selenocysteine is employed.

In one embodiment of the present invention, crystals of decarbamylase having an amino acid sequence of SEQ ID NO.: 1 or 2 may be grown in precipitant solution containing polyethylene glycol (PEG) or methoxypolyethylene glycol (PEGMME), a buffer agent, and any additive salt while carefully adjusting the concentration and pH of the solution within a predetermined range (the decarbamylase concentration is 1-50 mg/ml, the polyethylene glycol or methoxypolyethylene glycol concentration is 5-30 wt%, and pH is 6.0-9.0). Any of three kinds of basic technologies commonly used for growth of protein crystal, i.e., a vapor diffusion method, a dialysis method, and a batch method (Methods in Enzymology, Vol. 114, Diffraction Methods for Biological Macromolecules Part A or Vol. 276, Macromolecular Crystallography Part A), may be employed. The vapor diffusion method is preferable.

According to the vapor diffusion method, a droplet of a protein solution containing a precipitant is placed in a container holding a buffer (external solution) containing a high concentration of precipitant, and the container is sealed and allowed to stand. There are two methods for placing the droplet: a hanging-drop method and a sitting-drop method. In the hanging-drop method, a small droplet of protein solution is placed on a cover glass, and the cover glass is reversed in a reservoir so as to seal. In the sitting-drop method, an appropriate droplet stage is installed within the reservoir, a small droplet of protein solution is placed on the droplet stage, and the reservoir is sealed with a cover glass or the like. The solution within the reservoir contains a precipitant while a small amount of precipitant is present in the small protein droplet. The precipitant solution used in the vapor diffusion method contains the following components: (a)PEG or PEGMME having a molecular weight of 4000-9000, and preferably an average molecular weight of 7500, and a concentration of 10-20 wt%, (b) sodium chloride, lithium chloride, or magnesium chloride, as an additive salt, having a concentration of 0.1-0.5 M (best results are obtained with 0.2 M lithium chloride), and (c) a sufficient amount of buffer agent to provide pH 6.5-8.0, and preferably pH 7.5. To this end, 0.05-0.1 M HEPES (Sigma, St Louis, MO, USA) can be used. Other buffer agents such as sodium phosphate, potassium phosphate, and tris(hydroxymethyl)aminomethane malate, may be used.

The term "batch method" refers to a method in which a precipitant solution is added in small batches to a protein solution; when the solution is turned slightly cloudy, insoluble matter was removed by centrifugation; and thereafter, the supernatant is transferred to a small test tube which is in turn sealed and allowed to stand. The term "dialysis method" refers to a method in which a protein solution is subjected to dialysis using a semi-permeable membrane in a buffer containing a precipitant (external solution) (Methods in Enzymology, Vol. 114, Diffraction Methods for Biological Macromolecules Part A).

The term "predetermined size" as used herein refers to a minimum size which can be measured by X-ray crystallography. In the case of decarbamylase herein, it may be preferably 0.3x0.3x0.1 mm. The term "predetermined period of time" as used herein refers to a period of time sufficient for the size of crystal to reach a predetermined size or more. In the case of decarbamylase in the present invention, it may be preferably one day to three weeks.

The thus-prepared native crystal of decarbamylase (SEQ ID NO.: 1) having the predetermined size suited for X-ray crystallography has (1) an outer shape of a rhombic plate, and (2) a lattice constant which may be different among crystals having the same outer shape. When conditions, such as precipitant and buffer, are appropriately selected, small or micro crystals in a needle or columnar shape may be obtained though they are not suitable for X-ray crystallography. Concerning the use of such crystals, if a microcrystal of an enzyme is crosslinked by a protein crosslinking reagent such as glutaraldehyde, it is possible to maintain a stable enzyme reaction for a long time even when an organic solvent is used. This technique is called CLEC (Cross-Linked Enzyme Crystal) which has been reported (N. L. St. Clair & M. A. Navia, (1992) J. Am. Chem. Soc. 114, 7314-7316). The decarbamylase crystal of the present invention extends the range of application of the CLEC technique.

In one embodiment of the present invention, a heavy atom derivative crystal useful for X-ray crystallography, i.e., a crystal in which heavy metal atoms are coupled with proteins in the crystal while the crystallinity of native crystal is maintained, is provided. The heavy atom derivative crystal is utilized when using a heavy atom isomorphous replacement method and a multi-wavelength anomalous dispersion method which are basic techniques for protein X-ray crystallography. The heavy atom derivative crystal of decarbamylase may be prepared by a method of soaking native crystal in a solution which contains a heavy metal compound of an amount that provides a required concentration and in which the native crystal can be stably maintained for at least several days or more without dissolution and decay. Examples of heavy metal compounds used in the soaking method include metal salts or organic metal compounds containing gold, platinum, iridium, osmium, mercury, lead, uranium, samarium, etc. In the heavy metal soaking method, a stock solution which has a concentration of 0.1-100 mM of heavy metal compound, such as EMTS (ethyl mercury thiosalicylate sodium) which is a mercury compound, and potassium dicyanoaurate (I), and which has an appropriate composition of a precipitant and an additive salt which provides a desired pH. A preferable example of the stock solution is 0.01 MHEPES buffer (pH 7.5) containing 20-30 wt% polyethylene glycol 6000 and 0.2 M lithium chloride. Whether or not heavy metal atoms are introduced into a crystal (i.e., coupled with proteins in the crystal) is judged by collecting X-ray diffraction intensity data of a crystal prepared with the soaking method and comparing the data with the diffraction intensity data of a previously obtained native crystal.

Alternatively, a microorganism which produces decarbamylase (e.g., recombinant E. coli having DNA of the genus *Agrobacterium*) may be grown and cultured in medium containing selenomethionine or selenocysteine containing selenium which is a heavy metal atom, so that a mutant in which the methionine or cysteine residue of decarbamylase is substituted with selenomethionine or selenocysteine may be obtained. If the decarbamylase into which heavy metal atoms are introduced can be thus obtained without the soaking method, a heavy atom derivative crystal can be prepared by crystallization under the above-described conditions.

It is generally known that protein crystals suffer much damage from X-rays. Therefore, it is important to obtain crystal which resists damage in order to successfully perform X-ray crystallographic analysis. Recently, an attempt has been made to obtain high-quality and high-resolution diffraction data by freezing crystals and measuring the crystal when it is frozen (Methods in ENZYMOLOGY, Vol. 276, Macromolecular Crystallography, Part A, edited by C.W. Carter, Jr. and R.M. Sweet, [13] Practical Cryocrystallography (D.W. Rodgers)). In general, when a protein crystal is frozen, the protein crystal is treated with a solution containing a stabilizing agent for freezing such as glycerol for the purpose of preventing decay of the crystal due to freezing. In the present invention, the native crystal of decarbamylase and the frozen heavy atom derivative crystal may be prepared by soaking a small crystallized droplet or a crystal removed from an soaking solution directly into liquid nitrogen to freeze instantaneously without addition of a stabilizing agent for freezing. Alternatively, a frozen crystal may be prepared by subjecting a crystal soaked in a stock solution containing a stabilizing agent for freezing added, to the above-described instantaneous freeze procedure.

For novel proteins such as decarbamylase, since the stereostructure of similar proteins is not known, it is impossible to conduct structural analysis by a molecular replacement method which utilizes the stereostructure of the similar proteins. The stereostructure of such novel proteins may be determined by a heavy atom isomorphous replacement method (Methods in ENZYMOLOGY, Vol. 115, Diffraction Methods for Biological Macromolecules, Part B, edited by H.W. Wyckoff, C.H.W. Hirs, and S.N. Timasheff; and Methods in ENZYMOLOGY, Vol. 276, Macromolecular Crystallography, Part A, edited by C.W. Carter, Jr. and R.M. Sweet) or a multi-wavelength anomalous dispersion method (Edited by S.N. Timasheff, and Methods in ENZYMOLOGY, Vol. 276, Macromolecular Crystallography, Part A, edited by C.W. Carter, Jr. and R.M. Sweet). Specifically, electron density for calculating an initial phase is calculated from the difference in diffraction intensity among the diffraction data of a native crystal and a heavy atom derivative crystal, or the difference in diffraction intensity among diffraction data measured at different wavelengths. When the stereostructure of decarbamylase is determined with the heavy atom isomorphous replacement method or the multi-wavelength anomalous dispersion method, a heavy atom derivative crystal containing mercury, gold, platinum, uranium, or selenium atom, etc. as a heavy metal atom may be used. Preferably, a heavy atom derivative crystal obtained by a soaking method using the mercury compound EMTS or potassium dicyanoaurate (I) may be used.

The diffraction data of a native crystal and a heavy atom derivative crystal may be obtained using a beam line for of protein crystal structural analysis from R-AXIS IIc (Rigaku Corporation) or SPring-8 (Nishiharima large synchrotron radiation facility). To apply a multi-wavelength anomalous dispersion method, diffraction data of multiple wavelength may be obtained using a beam line for structural analysis of protein crystal of SPring-8. The obtained diffraction image data is processed by a data processing program attached to R-AXIS IIc or the program DENZO (Mcscience) or other similar image processing programs (or software for single crystal analysis) into reflection intensity data. Based on the reflection intensity data of a native crystal and the reflection intensity data at multiple wavelengths of a heavy atom derivative crystal, the positions of heavy metal atoms coupled with proteins in the crystal are calculated using a difference Patterson map and, thereafter, the program PHASES (W. Furey, University of Pennsylvania or CCP4 (British Biotechnology & Biological Science Research Counsil, SERC) or other similar diffraction data analysis programs are used to determine an initial phase where more precise heavy atom position parameters are employed. The resultant initial phase is improved to a more reliable phase by performing phase extension calculations gradually from low resolution to high resolution in accordance with a solvent flattening method and a histogram matching method using the program DM (CCP4 package) or a similar phase improving program (electron density improving program), where the solvent region of decarbamylase crystal is 30-50%, and preferably 35%. In a protein crystal, solvent molecules (mostly water molecules) other than the protein account for 30-60% of the volume. A volume occupied by solvent molecules in a solution is herein referred to as a "solvent region". In general, when a resultant protein crystal has non-crystallographic symmetry, it is possible to increase the reliability of the phase by performing electron density averaging which is called non-crystallographic symmetry (NCS) averaging. It has been known that the crystal of decarbamylase contains two molecules in a nonsymmetric unit from the results of measurement of the density of the crystal. It is inferred that the decarbamylase molecule in a crystal has a noncrystallographic two-fold axis. Based on an electron density map calculated using a phase after applying a solvent flattening method and refined heavy atomic coordinates, a non-crystallographic symmetry matrix containing translation and rotation is calculated. Meanwhile, a region in which protein molecules called a mask exist is identified from the electron density map obtained by the solvent flattening method. NCS averaging calculation is performed with the program DM or the like using a non-crystallographic symmetry matrix and a mask, thereby obtaining a more reliable phase and an electron density map used for construction of the stereostructure model.

A stereostructure model of decarbamylase may be constructed with Program O (A. Jones, Uppsala Universitet, Sweden) based on the electron density map displayed on three-dimensional graphics in accordance with the following procedure. Firstly, a plurality of regions having characteristic amino acid sequences (e.g., a partial sequence containing a tryptophan residue) are found on the electron density map. Next, a partial structure of an amino acid residue corresponding to its electron density is constructed using Program O on the three-dimensional graphics while referencing the amino acid sequence where the found regions are used as starting points. This process is sequentially repeated, thereby matching all amino acid residues of decarbamylase to respective electron density. As a result, the initial stereostructure model of the whole molecule is constructed. The constructed stereostructure model, which is used as a starting model structure, is refined in accordance with a refinement protocol for a structural refinement program XPLOR (A.T. Brunger, Yale University), so that three-dimensional coordinates describing the stereostructure are refined. The stereostructure of the native crystal of decarbamylase (e.g., decarbamylase as shown in SEQ ID NO.: 1) and the stereostructure of the native crystal of a decarbamylase mutant (e.g., a decarbamylase mutant having a sequence shown in SEQ ID NO.: 2) may be determined by calculating the initial phase by the molecular replacement method using the stereostructure of the resultant EMTS derivative crystal, and conducting the above-described procedures, i.e., the electron density improvement, the model construction, and the structural refinement. In this manner, the determination of the stereostructure of decarbamylase according to the present invention is completed. The thus-determined stereostructure of decarbamylase may be compared with the stereostructure of various proteins (including enzymes having a function similar to that of decarbamylase) registered in the Protein Data Bank (PDB) which is a public databank for the stereostructure of proteins. The stereostructure of N-carbamyl-sarcosine-amidohydrolase which catalyzes a decarbamylation reaction similar to that of decarbamylase (Protein Data Bank ID: 1NBA) is known. However, the stereostructure of decarbamylase is not recognized as having structural similarity to the stereostructure of this enzyme. Among the proteins whose stereostructure is registered in the Protein Data Bank, penicillin acylase (1PNK), glucosamine-6-phosphate synthetic enzyme (1GDO), glutamine phosphoribosylpyrophosphate amidotransfelase (1ECF), proteosome (1PMA), etc. have a so-called four-layer sandwich structure, in which a helices are in close contact with the opposite sides of a sandwich of two β sheets in a substructure of the stereostructure called a domain, indicating the similarlity to the stereostructure of decarbamylase. However, these domain structures are different from the stereostructure of decarbamylase in the topological arrangement of α helices and β strands, i.e., protein spatial structural topology (T.P. Flores et al., (1994), Prot. Eng. 7, 31-37). In other words, the stereostructure of decarbamylase is characterized by parallel β strands in a β sheet structure (Figure **1**). Further, decarbamylase has a novel stereostructure in terms of the four-layer sandwich structure. Thus, the term "topology" as used herein refers to the arrangement or spatial configuration of the secondary structural units of a protein.

The term "α helix" as used herein refers to a helical structure which is one of the secondary structures of proteins or peptides and one of the most energetically stable structures in which every 3.6 amino acid residues constitute one turn which spans a pitch of 5.4 Å. Examples of amino acids which are likely to form an α helix include glutamic acid, lysine, alanine, and leucine. Conversely, examples of amino acids which are unlikely to form an a helix include valine, isoleucine, proline, and glycine. The term "β sheet" as used herein refers to one of the secondary structures of protein or polypeptide in which two or more polypeptide strands having a zigzag conformation are arranged in parallel in the shape of a sheet where the amido groups and carbonyl groups of the peptides and the carbonyl groups and amido groups of the neighboring peptides form hydrogen bonds, respectively, and which is energetically stable. The term "parallel β sheet" refers to a β sheet of the type that the amino acid sequences of adjacent polypeptide strands have the same directions. The term "antiparallel β sheet" refers to a β sheet of the type that the amino acid sequences of adjacent polypeptide strands have the opposite directions. The term "β strand" as used herein refers to a single peptide strand constituting a β sheet, which has a zigzag conformation.

It was revealed that the carboxy-terminal region of decarbamylase containing about 30 residues (an amino acid at about position 280 to the carboxy terminus at position 303 in SEQ ID NO.: 1 or 2) is involved in formation of a dimer through intermolecular interaction. Further, in the decarbamylase crystal subjected to this study, the dimers form tetramers due to intermolecular interactions. The four-layer sandwich structure of decarbamylase comprises a secondary structural unit consisting of four α helices and twelve β strands. Table 1 shows all secondary structural units.

**Table 1**

| Secondary Structure Units of Decarbamylase | |
|---|---|
| α helix | β strand |
| Arg20 -Arg36 | Gln3 -Gln10 |
| Glu61 -Asp65 | Phe41 -Yal43 |
| Arg78 -Leu87 | Gly90 -Yal199 |
| Glu145-Tyr148 | Arg106-Val114 |
| Pro177-Leu185 | Ile120-Arg125 |
| Thr211-Asn226 | Val158-Val161 |
| Arg279-Arg282 | Ala164-Met168 |
| | Ile190-Tyr195 |
| | Trp229-Gly234 |
| | Gly237-Glu239 |
| | Cys242-Leu244 |
| | Cys249-Val251 |
| | Ile257-Leu260 |
| | Glu267-Asp274 |

Characteristics of the stereostructure of decarbamylase of the present invention will be summarized below.
(1) A structure called a four-layer sandwich in which two a helices are closely in contact with each of the opposite sides of a sandwich of two β sheets consisting of six β strands (Figures **1** and **2**). In Figure 1, four α helices at the first, third, fifth, and sixth positions from the amino terminus are closely in contact with the β sheets (hereinafter referred to as α1, α3, α5, and α6).
(2) A β sheet essentially consists of six parallel β strands and has an orientation which has not been observed in proteins whose stereostructure is known (Figure **1**).
(3) As amino acid residues which catalyze enzyme reactions, one cysteine residue, two glutamic acid residues, one lysine residue, one histidine residue, and two arginine residues are contained. An active site is present which has a substrate binding manner (Figure **3**) which is not possessed by enzymes whose stereostructure is known (Figure **4**).
(4) The carboxy-terminal region of about 30 residues plays a role in formation of a dimer through intermolecular interaction.

The specific coordinate data of the above-described stereostructure has been registered in the Protein Data Bank (PDB: run by The Research Collaboratory For Structual Bioinformatics (RCSB)) as Compound: N-Carbamyl-D-Amino Acid Amidohydrolase, Exp. Method: X-ray Diffraction (Acc. No.: 1ERZ). The data is herein incorporated by reference.

The stereostructure determination of decarbamylase of the present invention has been completed, whereby amino acid residues of the enzyme involved in catalytic activity can be inferred; not only the stereostructure of the enzyme alone but also the stereostructure model of a complex obtained by the enzyme being bound to a substrate (e.g., D-N-carbamoyl-hydroxyphenylglycine) can be easily constructed by a molecular modeling technique (Swiss-PDBViewer (above), Autodock (Oxford Molecular), Guex, N. and Peitsch, M.C. (1997) SWISS-MODEL and the Swiss-PDBViewer: An environment for comparative protein modeling, Electrophoresis 18, 2714-2723; Morris, G.M et al., J. Computational Chemistry, 19:1639-1662, 1998; Morris, G.M. et al., J. Computer-Aided Molecular Design, 10, 294-304, 1996; Goodsell, D.S. et al., J. Mol. Recognition, 9:1-5, 1996). With the stereostructure and the stereostructure model, a structure relating to amino acid residues present at an active site for a catalytic reaction and the reaction mechanism of the active site can be found to some extent. The active site of decarbamylase is formed of a cavity comprising amino acid residues Glu46, Lys126, His143, Glu145, Cys171, Arg174, and Arg175 (Figure **4**). The analysis of conserved amino acid residues found by comparison in amino acid sequence with enzymes having weak sequence similarities with decarbamylase, such as amidase and nitrilase, suggests that Glu46, Lys126, Glu145, and Cys171 are strongly involved in the catalytic reaction. In particular, Cys171 is expected to be a catalytic residue essential to formation of an acyl intermediate which is an intermediate for the enzyme reaction (Figure 3), suggesting that decarbamylase is a cysteine hydrolase. This finding is in agreement with the experimental fact that a mutation from Cys171 to Ser171 causes a loss of catalytic activity (R. Grifantini et al., (1996), J. Biol. Chem. 271, 9326-9331). The role of Arg174 and Arg175 is considered to be to contribute to stabilization of the carboxyl group of the D-N-α-carbamyl amino acid substrate due to electrostatic interaction.

Based on these findings from the stereostructure, modifications of decarbamylase can be designed for the purpose of improving the stability or enzymatic activity of decarbamylase. The term "stability" of an enzyme as used herein refers to that even after the enzyme is denaturated at a temperature (e.g., 70°C) higher than usual environment for living organisms, the enzymatic activity remains at least 10% compared with before the thermal denaturation, preferably at least 25%, more preferably at least 50%, even more preferably at least 80%, and most preferably at least 90%. An improvement in stability may be measured by ΔTm (difference in denaturating temperature). The term "enzymatic activity" as used herein refers to an activity to convert D-N-carbamoyl-α-amino acid to corresponding D-α-amino acid when decarbamylase is mentioned.

The term "design method" or "molecular design technique" for a mutant molecule as used herein refers to that the amino acid sequence and the stereostructure of a protein or a polypeptide molecule before mutation (e.g., naturally-occurring molecule) are analyzed to predict characteristics (e.g., catalytic activity and interactions with other molecules) of each amino acid; and amino acid mutations appropriate for desired modifications of characteristics (e.g., an improvement in catalytic activity and an improvement in protein stability) are calculated. The design method is preferably performed using a computer. Examples of computer programs used in the design method include as mentioned herein: X-ray diffraction data processing program DENZO (McScience) as a program for analyzing the structure; PHASES (Univ. of Pennsylvania, PA, USA) as a program for determining the phase; program DM (CCP4 package, SERC) as a program for improving the initial phase; program O (Uppsala Universitet, Uppsala, Sweden) as a program for obtaining three-dimensional graphics; XPLOR (Yale University, CT, USA) as a program for refining the stereostructure; and Swiss-PDBViewer (above) for a program for mutation introduction modeling.

Examples of amino acid mutation as used herein for design of mutants include amino acid addition, deletion, or modification as well as amino acid substitution. Amino acid substitution refers to that one or more amino acid (e.g., 1-20 amino acids, preferably 1-10 amino acids, and more preferably 1-5 amino acids) of an original peptide are substituted with the same number of amino acids. Amino acid addition refers to that one or more amino acid (e.g., 1-20 amino acids, preferably 1-10 amino acids, and more preferably 1-5 amino acids) are added to an original peptide chain. Amino acid deletion refers to that one or more amino acid (e.g., 1-20 amino acids, preferably 1-10 amino acids, and more preferably 1-5 amino acids) are deleted from an original peptide. Amino acid modification includes, but is not limited to, amidation, carboxylatlon, sulfation, halogenation, alkylation, glycosylation, phosphorylation, hydroxylation, acylation (e.g., acetylation), etc. An amino acid to be substituted or added may be a naturally-occurring amino acid or a non-naturally-occurring amino acid, or an amino acid analog. A naturally-occurring amino acid is preferable.

The term "naturally-occurring amino acid" refers to the L-isomer of a naturally-occurring amino acid. Examples of the naturally-occurring amino acid refer to glycine, alanine, valine, leucine, isoleucine, serine, methionine, threonine, phenylalanine, tyrosine, tryptophan, cysteine, proline, histidine, asparticacid, asparagine, glutamicacid, glutamine, γ-carboxyglutamic acid, arginine, ornithine, and lysine. Unless otherwise mentioned, all amino acid are herein L-isomers.

The term "non-naturally-occurring amino acid" refers to an amino acid which is not usually found in naturally-occurring proteins. Examples of the non-naturally-occurring amino acid include norleucine, paranitrophenylalanine, homophenylalanine, parafluorophenylalanine, 3-amino-2-benzylproprionic acid, D- or L-homoarginine, and D-phenylalanine.

The term "aminoacid analog" refers to a molecule which is not an amino acid but has a physical property and/or function similar to those of an amino acid. Examples of the amino acid analog include ethionine, canavanine, 2-methylglutamine, etc.

In another embodiment of the present invention, a decarbamylase mutant may be in the form of a peptide salt, such as ammonium salt (alkyl or aryl ammonium salt), sulfate, hydrogen sulfate, phosphate, hydrogen phosphate, dihydrogen phosphate, thiosulfate, carbonate, bicarbonate,benzonate, sulfonate, thiosulfonate, mesylate (methylsulfonate), ethylsulfonate, and benzenesulfonate.

Hereinafter, amino acid mutations in a protein for producing a mutant will be discussed. Methods for amino acid substitution and the like include, but are not limited to, changing a codon, which is a DNA sequence encoding an amino acid, in techniques utilizing chemical synthesis or genetic engineering.

A certain amino acid may be substituted with another amino acid in a protein structure, such as a cationic region or a substrate molecule binding site, without a clear reduction or loss of interactive binding ability. A certain biological function of a protein is defined by the interactive ability or property of the protein. Therefore, a particular amino acid substitution may be performed in an amino acid sequence, or at the DNA code sequence level, to produce a protein which maintains the original property after the substitution. Therefore, various modifications of peptides as disclosed herein and DNA encoding such peptides may be performed without clear losses of biological usefulness.

When the above-described modifications are designed, the hydrophobicity indexes of amino acids may be taken into consideration. The hydrophobic amino acid indexes play an important role in providing a protein with an interactive biological function, which is generally recognized in the art (Kyte. J and Doolittle, R.F., J. Mol. Biol. 157(1):105-132, 1982). The hydrophobic property of an amino acid contributes to the secondary structure of a generated protein and then regulates interactions between the protein and other molecules (e.g., enzymes, substrates, receptors, DNA, antibodies, antigens, etc.). Each amino acid is given a hydrophobicity index based on the hydrophobicity and charge properties thereof as follows: isoleucine (+4.5); valine (+4.2); leucine (+3.8); phenylalanine (+2.8); cysteine/cystine (+2.5); methionine (+1.9); alanine (+1.8); glycine(-0.4); threonine (-0.7); serine (-0.8); tryptophan (-0.9); tyrosine (-1.3); proline (-1.6); histidine (-3.2); glutamic acid (-3.5); glutamine (-3.5); aspartic acid (-3.5); asparagine (-3.5); lysine (-3.9); and arginine (-4.5)).

It is well known that if a certain amino acid is substituted with another amino acid having a similar hydrophobicity index, a resultant protein may still have a biological function similar to that of the original protein (e.g., a protein having an equivalent enzymatic activity). For such an amino acid substitution, the hydrophobicity index is preferably within ±2, more preferably within ±1, and even more preferably within ±0.5. It is understood in the art that such an amino acid substitution based on the hydrophobicity is efficient. As described in US Patent No. 4,554,101, amino acid residues are given the following hydrophilicity indexes: arginine (+3.0); lysine (+3.0); aspartic acid (+3.0±1); glutamic acid (+3.0±1); serine (+0.3); asparagine (+0.2); glutamine (+0.2); glycine (0); threonine (-0.4); proline (-0.5±1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); and tryptophan (-3.4). It is understood that an amino acid may be substituted with another amino acid which has a similar hydrophilicity index and can still provide a biological equivalent. For such an amino acid substitution, the hydrophilicity index is preferably within ±2, more preferably ± 1, and even more preferably ±0.5.

The term "conservative substitution" as used herein refers to amino acid substitution in which a substituted amino acid and a substituting amino acid have similar hydrophilicity indexes or/and hydrophobicity indexes. Examples of the conservative substitution include, but are not limited to, substitutions within each of the following groups: arginine and lysine; glutamic acid and aspartic acid; serine and threonine; glutamine and asparagine; and valine, leucine, and isoleucine, which are well known to those skilled in the art.

Information on the mechanism of a catalytic reaction obtained from the stereostructure is very useful for molecular design aiming for an improvement in the specific activity of an enzyme and optimization of the optimum pH of a reaction. In the present invention, by changing the pKa of the side chain sulfide (SH) group of Cys171 of decarbamylase, an amino acid mutation which leads to changes in the specific activity and the optimum pH can be designed. Specifically, the effect of the amino acid mutation on the pKa of the SH group can be estimated by calculating the electrostatic potential of a protein (Takahashi et al., (1992), Biopolymers 32, 897-909), thereby making it possible to design an appropriate amino acid mutation. For example, a mutation which causes the electrostatic field around the sulfur atom of Cys171 to be more positive, promotes detachment of the thiol group (SH) of Cys171, and has substantially no influence on the electrostatic field around the side chain carboxy group of Glu46 and/or Glu145, is preferable.

In view of the stereostructure of decarbamylase of the present invention, it is inferred that five cysteine residues present in decarbamylase are involved in the function and physical properties thereof, such as catalytic activity and resistance to air oxidation. Concering the role of a cysteine residue of decarbamylase, a different bacterium *(Agrobacterium radiobacter* NRRL B11291) having a high level of sequence homology with decarbamylase of the above-described bacterium *(Agrobacterium* sp. KNK712 (Kaneka bacterium)), has been studied by combinations of amino acid mutations, denaturing agents, and Cys modification agents (R. Grifantini et al., (1996), J. Biol. Chem. 271, 9326-9331). In this study, the five cysteine residues are not involved in the formation of disulfide bonding, and the catalytic activity of decarbamylase derived from A. radiobacter NRRL B11291 is lost by substitution of Cys172 corresponding to Cys171 to Ser but not affected by the other cysteine mutations. It is inferred that Cys171 is essential to the catalytic activity. This is also strongly suggested by the fact that a corresponding cysteine residue is conserved in other similar enzymes. The term " corresponding amino acid" as used herein refers to an amino acid of a certain protein molecule or polypeptide molecule which has (or is expected to have) a function similar to that of a predetermined amino acid in a protein or peptide which is used as a comparison reference and, particularly in the case of enzyme molecules, an amino acid which is present in a similar position in an active site and has a similar contribution to a catalytic activity.

In decarbamylase having amino acid sequences of SEQ ID NO.: 1 and 2, it is inferred that Cys192 and Cys249 are unlikely to be modified and are buried within the molecule, and Cys242 and Cys278 are easily modified chemically and positioned at the loop structure portion on the molecule surface. By substituting two cysteine residues (Cys243 and Cys279) of A. radiobacter NRRL B11291 corresponding to Cys242 and Cys278 of SEQ ID NO.: 1 with alanine, a mutant having improved stability has been obtained (Japanese Laid-Open Publication No. 8-84584). As described above, Cys242 and Cys278 are confirmed to be present on the molecule surface from the stereostructure, and it is suggested that these Cys residues are involved in the resistance of decarbamylase to air oxidation. It is inferred that Cys192 and Cys249 buried within the molecule do not contribute much to the improvement of the resistance to air oxidation. Nevertheless, by substituting Cys192 and Cys249 with hydrophobic amino acid residues having a large volume so as to compensate for the side chain volume or the cavity in the molecule, a resultant decarbamylase mutant can have improved stability against heat or organic solvents.

It may be considered that not only cysteine residues but also methionine residues are involved in a reduction in the enzymatic activity of decarbamylase due to air oxidation and in the conservation of stability. According to the stereostructure of decarbamylase of the present invention, out of nine methionine residues in decarbamylase, there are five residues which are fully buried within the molecule, while there are two residues which are fully exposed to the outside of the molecule (Met238 and Met243) and which are present at the turn structure portion. It is inferred that these two residues are much more involved in the resistance of decarbamylase to air oxidation. The term "turn structure" or "β turn structure" as used herein refers to a local structure consisting of three or more amino acids in which the extending direction of a peptide principal chain is largely changed between the secondary structures in the stereostructure. Further, Met4 and Met72 are located around the molecule surface and substantially buried within the molecule, but is positioned at a site which causes them to easily contact solvent molecules and the like. Therefore, these are likely to be involved in the resistance of decarbamylase to air oxidation. Based on these structural findings, other amino acid substitutions by which the resistance of decarbamylase to air oxidation is expected to be increased can be designed. Specifically, Met4 andMet72 are substituted with hydrophobic amino acid residues which compensate for the side chain volume or the cavity in the molecule and do not suffer from air oxidation, so that the resultant mutant can have improved resistance. Met238 and Met243 are fully exposed to the outside of the molecule. Therefore, preferably, by substituting these residues with neutral or hydrophilic amino acids which do not suffer from air oxidation, the resistance can be improved. Further, the turn structure portion of decarbamylase ranging from Met238 to Met243 contains Cys242 which is susceptible to air oxidation. By producing a triple mutant in which these three residues are substituted with appropriate amino acids, or producing a mutant in which the turn structure containing the three residues are deleted, the resistance to air oxidation can be improved. Alternatively, by replacing the sequence of the turn structure containing these residues with another protein β turn structure, the resistance to air oxidation can be improved. When designing cysteine and methionine mutants, an optimum amino acid can be selected by substituting these residues with other amino acids on a computer to analyze the structural energy in terms of the stability of the mutants.

Other than cysteine and methionine residues, a design which focuses on generally known factors in the stability of a protein may be conducted, such as amino acid mutation which compensates for the cavity in the molecule (e.g., the cavity formed around an amino acid Asn92 of decarbamylase), mutation of amino acids having disadvantageous conformation in terms of energy (e.g., amino acids Pro203 and Val236 of decarbamylase), and stabilization of an α helix structure.

As a design strategy different from the above-described modification design by amino acid mutations, a region inferred not to be involved in the enzymatic activity of decarbamylase is deleted to modify decarbamylase to a lower molecular weight enzyme, thereby making it possible to improve the stability. For example, a part or entirety of a loop structural region away from an active site may be deleted, resulting in a more closely packed decarbamylase. Further, the inventors of the present invention infer that a region of decarbamylase having about 30 residues from the carboxy terminus is involved in formation of a dimer. It is inferred that when decarbamylase exhibits catalytic capability in a general buffer, the decarbamylase is present in the form of a dimer or tetramer. It is inferred that a monomer protein is more advantageous in terms of a recovering process from the denatured state of the protein. If the dimer formation is inhibited by deleting a part or entirety of the carboxy-terminal region of about 30 residues, a stable monomer can be prepared. From an industrial viewpoint, is utilized as a so-called immobilized enzyme where the decarbamylase is immobilized on a carrier. Also, in this case, a monomer can be more efficiently immobilized.

The decarbamylase mutant of the present invention may be designed or prepared by a number of methods other than the above-described methods. For example, the sequence of an enzyme having decarbamylase activity may be mutated by oligonucleotide-specific mutagenesis or other conventional technical means (deletion) at the sites identified as being desired for mutations using the present invention. Alternatively, decarbamylase mutants may be prepared by site-specific substitution of a specific amino acid with a non-naturally-occurring amino acid. For example, decarbamylase mutants may be prepared by substitution of a specific cysteine ormethionine residue with selenocysteine or selenomethionine. This may be achieved by growing a host living organism capable of expressing either a wild-type polypeptide or a mutant polypeptide on growth medium which either (or both) naturally-occurring cysteine or methionine has been exhausted, and either (or both) selenocysteine or selenomethionine has been enriched. When a homologous recombinant method is used, mutation is introduced into a DNA sequence encoding decarbamylase using a synthetic oligonucleotide. Such an oligonucleotide contains a nucleotide sequence adjacent to a desired mutation site. Mutation may be prepared in the full-length DNA sequence of decarbamylase or any sequence encoding a fragment polypeptide thereof.

The DNA sequence of mutated decarbamylase produced by the above-described method or an alternative known method in the art in accordance with the present invention may be expressed using an expression vector. As is well known in the art, the expression vector typically contains elements which enable self replication in a host cell independent of the host genome, and one or more phenotype markers for screening. Before or after insertion site of a DNA sequence surrounding a desired decarbamylase mutant encoding sequence, the expression vector also contains a promoter, an operator, a ribosome binding site, a translation initiating signal, and, if necessary, a repressor gene or various activator genes, and a regulatory sequence encoding a stop signal. In some embodiment, when a produced mutant is desired to be secreted, nucleotides encoding a "signal sequence" may be inserted before the decarbamylase mutant encoding sequence. In order to be expressed under the control of regulatory sequences, a desired DNA sequence must be operatively linked to the regulatory sequences. Specifically, the decarbamylase mutant encoding sequence under the control of the regulatory sequences must have an initiation signal (i.e., ATG) to maintain an appropriate reading frame for the purpose of enabling this sequence to produce an expression product.

Any of a wide range of available, well known expression vectors is useful for expression of a sequence encoding the mutated decarbamylase of the present invention. These include known bacterial plasmids (e.g., pBR322), a more wide host range of plasmids (e.g., RP4, phage DNA), 2µ plasmid, or yeast plasmids as derivatives thereof, and vectors consisting of fragments of a chromosomeal DNA sequence, a nonchromosomeal DNA sequence, and a synthetic DNA sequence, such as a vector obtained by a combination of a plasmid and phage DNA.

Any of a wide range of regulatory sequences which regulate the expression of a DNA sequence when operatively linked to the DNA sequence can be used in a vector. Examples of such useful expression regulatory sequences include other sequences known to regulate viral gene expression and a combination thereof.

A wide range of species of hosts are useful for production of the decarbamylase mutant of the present invention. Examples of such hosts include bacteria (e.g., E. coli, Bacillus, and Streptomyces), fungi (e.g., yeast), animal cells (e.g., a CHO cell), plant cells, and transgenic host cells.

It should be understood that not all expression vectors and systems function in the same manner for expression of the mutated DNA sequence of the present invention to produce the decarbamylase mutant. Not all hosts function equally well in the same expression system. Nevertheless, it is possible for those skilled in the art to select appropriate vectors, expression regulatory sequences, and hosts without conducting undue experimentation or departing from the scope of the present invention.

For example, the replication ability of the vector must be taken into consideration in screening. Various factors must be taken into consideration in screening expression regulatory sequences. Examples of these include the relative strength of a system, the regulatory ability, and the compatibility with a DNA sequence encoding the decarbamylase mutant of the present invention. Particularly, the compatibility with a potential secondary structure should be taken into consideration. A host should be selected by considering the compatibility with a selected vector, the toxicity of the decarbamylase mutant to the host, the ability to secret mature products, the ability to fold a protein and the ability to form an appropriate higher-order structure, fermentation requirements, ease of purifying the decarbamylase mutant from the host, and safety. Among these parameters, those skilled in the art can select various combination of vector/expression regulatory system/host, which can produce a useful amount of mutated decarbamylase.

Decarbamylase mutants produced by these systems or other systems may be purified by various conventional processes for purifying naturally-occurring enzymes having decarbamylase activity.

Once a mutation to decarbamylase is created at a desired position (e.g., an active site, a site involved in stability, or a binding site, etc.), the resultant mutant may be subjected to testing on any of intended several characteristics.

For example, mutants may be screened with respect to a change in electric charge at a physiological pH. The charge change is determined by measuring isoelectric points (pI) of a decarbamylase mutant while comparing these with the isoelectric points before mutation. Isoelectric points are measured by gel electrophoresis in accordance with a method described in Wellner, D., Analyt. Chem., 43, p. 597 (1971). A decarbamylase protein having a changed surface charge is a mutant having a pI changed by a substituting amino acid positioned on the surface of the enzyme, as provided by the structural information of the present invention.

Further, mutants may be screened for high specific activity compared with native decarbamylase. The activity of mutant decarbamylase is determined by measuring its ability to convert D-N-carbamoyl-α-amino acid to D-α-amino acid using an assay as described herein (see Example 7 below), for example.

D-α-amino acids produced by utilizing the decarbamylase mutant of the present invention may be used as pharmaceutical intermediates (e.g., D-phenylglycine and D-para-hydroxyphenylglycine) to produce pharmaceuticals (e.g., synthetic penicillin and synthetic cephalosporin). A pharmaceutical composition containing the thus-produced pharmaceutical may also be provided according to the present invention. The pharmaceutical composition may contain a pharmaceutically acceptable auxiliary component including a excipient, a stabilizing agent, a carrier, etc.

D-α-amino acids produced by utilizing the decarbamylase mutant of the present invention may be used as pesticide intermediates (e.g., D-valine) to produce pesticides (e.g., fluvalinate). A pesticidal composition containing the thus-produced pesticide may also be provided according to the present invention. The pesticidal composition may contain an agriculturally acceptable auxiliary component including a excipient, a stabilizing agent, a carrier, etc.

D-α-amino acids produced by utilizing the decarbamylase mutant of the present invention may be used as intermediates (e.g., D-alanine and D-aspartic acid) of food additives to produce food additives (e.g., Alitame).

In another embodiment of the present invention, inhibitors of decarbamylase mutants or enzymes similar thereto may be designed and produced. Such inhibitors may be designed by utilizing the structural information of decarbamylase. Those skilled in the art can identify the inhibitors as being competitive, uncompetitive, or noncompetitive based on, for example, computer compatible enzyme reaction kinetics data employing standard formulas in Segel, I.H., Enzyme Kinetics, J. Wiley & Sons, (1975). Further, the inhibitors may be designed using information of reaction intermediates of decarbamylase or enzymes similar thereto (e.g., the stereostructure of a complex with a substrate or reaction product). Such information may be useful for design of improved analogs of known compounds which serve as inhibitors of known decarbamylase or enzymes thereto, and a novel class of inhibitors.

In a still another embodiment of the present invention, the stereostructure of decarbamylase of the present invention may be utilized to modify polypeptide enzymes or protein enzymes (e.g., amidase or nitrilase) having an amino acid sequence similar to that of decarbamylase. Such modification may be conducted in a manner similar to that for the above-described mutant design. The "similar polypeptide enzyme or protein enzyme" preferably has an amino acid sequence which is identical to the amino acid sequence of decarbamylase represented by SEQ ID NO. : 1 or 2 over the full-length amino acid sequence by representatively at least 30%, preferably at least 50%, and more preferably at least 80%, and particularly, with respect to a region defining the active site of decarbamylase (a range of amino acid number 38-49, 108-127, 143-148 and 164-177 including amino acids Glu46, Lys126, Glu145, and Cys171 in SEQ ID NO.: 1 or 2), representatively at least 60%, preferably at least 80%, more preferably 90%, and even more preferably 95%.

As an example of the transformation of a characteristic of a certain enzyme using the stereo structure of an enzyme similar thereto, there is a study in which the substrate specificity of an enzyme called expandase was transformed by utilizing the similar stereostructure of an isopenicillin synthetic enzyme. See International Publication WO97/02005. According to this document, the isopenicillin synthetic enzyme whose the stereostructure had already been determined (Roach(1995), Nature, 375, 700-704) and expandase which is similar to the synthetic enzyme in the primary structure (i.e., amino acid sequence) and had been suggested to have a similar stereostructure were compared with each other with respect to their sequences, thereby identifying the amino acid residues of expandase which are involved in recognition of a substrate, and by mutating these amino acid residues, the substrate specificity of expandase was transformed to produce a mutated enzyme which can act on penicillin G.

The comparison of identity of amino acid sequences may be calculated using, for example, the following sequence analyzing tools: FASTA (W.R. Pearson and D.J. Lipman, PNAS85, 2444-2448 (1988)); BLAST (S.F. Altschul, T.L. Madden, A.A. Schaffer, J, -H., Zhang, Z. Zhang, W. Miller, and D.J. Lipman (1997) Nucl. Acids. Res. 25:3389-3402 (1997)); Unix-base GCG Wisconsin Package (Program Manual for the Wisconsin Package, Version 8, September 1994, Genetics Computer Group, 575 Science Drive Madison, Wisconsin, USA 53711; Rice, P. (1996) Program Manual for EGCG Package, PeterRice, The Sanger Centre, Hinxton Hall, Cambridge, CB101RQ, England) and the ExPASy World Wide Web molecular biology server (Geneva University Hospital and University of Geneva, Geneva, Switzerland) and MacVector 6.0 (Teijin System Technology).

According to the present invention, polypeptide or protein enzyme mutants having an amino acid sequence similar to that of decarbamylase may be provided by utilizing the above-described method. These enzyme mutants may be used instead of their wild-type enzymes in a bioreactor, for example.

In another aspect, the present invention provides a system for designing decarbamylase mutants using a computer. This system comprises means for determining the stereostructure of a crystal of an enzyme having decalbamylase activity by X-ray crystallography, and means for designing the decalbamylase mutants having improved physical property and/or function based on the determined stereostructure. The computer system of the present invention may be constructed using a computer system known in the art (e.g., the known systems in the art as described herein).

According to the present invention, a computer readable recording medium in which the three-dimensional coordinate data of the stereostructure of the above-described molecules such as decalbamylase and/or a program of a molecular designing and/or modifying method are recorded may also be provided. Examples of the computer readable recording medium include magnetic tape, magnetid disks (e.g., floppy disk), magneto-optical disks (e.g., MO), and optical disks (e.g., CD-ROM, CD-R, CD-RW, and DVD-ROM). In one embodiment, such a computer readable recording medium may be a computer recording medium in which a program for executing a design process of a decarbamylase mutant. In this case, the design process may comprise the steps of inputting data of a crystal of an enzyme having decalbamylase activity determined by X-ray crystallography and designing a decalbamylase mutant having improved physical property and/or function based on the determined stereostructure. In another embodiment, the present invention provides a recording medium in which data describes the stereostructure of a decarbamylase mutant. In this case, the stereostructure of the mutant may be obtained by a process comprising the steps of inputting data of a crystal of an enzyme having decalbamylase activity determined by X-ray crystallography and designing a decalbamylase mutant having improved physical property and/or function based on the determined stereostructure.

The present invention will be described in more detail in the following examples. These examples are provided for the purpose of illustrating the present invention, but are not intended to limit the present invention.

### (Examples)

Reagents used in the following examples were obtained from Nacalai Tesque, Wako Pure Chemical Industries, Ltd., or SIGMA (St Louis, MO, USA) unless otherwise mentioned.

### (Example 1) Preparation of Native crystal of Decarbamylase.

Decarbamylase was obtained by a known method (see H. Nanba et al., Biosci. Biotechnol. Biochem. 62, 875-881 (1998)). Decarbamylase solution (0.001 M HEPES buffer, pH 7.5) was prepared to a concentration of 10-20 mg/ml. 10 µl of the decarbamylase solution and 10 µl of 0.1 M HEPES buffer (pH 7.5, manufactured by SIGMA) as a precipitant containing 15-20 wt% polyethylene glycol 6000 (manufactured by Nacalai Tesque) and 0.2 M lithium chloride were mixed together on a droplet stage. After 300 µl of solution having the above-described precipitant composition was sealed as reservoir solution, crystallization was conducted by a vapor diffusion method at 20-25°C. About two days to two weeks after the start of the crystallization, a crystal was grown up to a size of 0.3x0.3x0.1 mm to 0.6x0.6x0.3 mm.

### (Example 2) Preparation of Heavy Atom Derivative Crystal of Decarbamylase

The native crystal obtained in Example 1 was removed from a small droplet on the droplet stage under a microscope. The native crystal was soaked in 0.1 M HEPES buffer (pH 7.5) containing 20 wt% polyethylene glycol 6000 (manufactured by Nacalai Tesque) and 0.2 M lithium chloride which was prepared to have a EMTS (sodium ethylmercurithiosalicylate) concentration of 0.5-1.0 mM (EMTS is a mercury compound) overnight to prepare a heavy atom derivative.

### (Example 3) Freeze of Decarbamylase Crystal

The native crystal and the heavy atom derivative crystal of decarbamylase prepared in Examples 1 and 2 were removed from the crystallized droplet and directly soaked in liquid nitrogen without a stabilizing agent for freezing such as glycerol.

### (Example 4) Collection of X-ray Diffraction Data of Decarbamylase Crystal

To conduct analyses by a multi-wavelength anomalous dispersion method, diffraction data was obtained in the radiation light facility Spring-8. Such measurement was conducted using the EMTS derivative crystal frozen in liquid nitrogen. For the measurement, three wavelengths (0.98, 1.00, and 1.27 Å) were used, taking anomalous dispersion of mercury into consideration. Three wavelength diffraction data were all collected from a piece of crystal. 53 frames of diffraction image data obtained were processed by a data processing program DENZO (McScience) and the result is shown in Table 2. The native crystal of decarbamylase was measured in the frozen form using 1.00 Å wavelength to obtain diffraction data and the results are shown in Table 3. See "X-sen Kaiseki Nyumon [Introduction to X-ray Analysis]" (Masao Kakuto et al., Tokyo Kagaku Dojin) for terms such as independent reflectivity, lattice constant, etc.

**Table 2**

| Results of Processing Diffraction Data of EMTS Derivative Crystal of Decarbamylase | | | | | |
|---|---|---|---|---|---|
| Wavelength | Resolution | R-merge | Yield | Independent reflection constant | Unit cell constant |
| (Å) | (Å) | (%) | (%) | | (Å) |
| 0.98 | 1.8 | 5.1 | 98.5 | 56679 | a=66.92 |
| | | | | | b=135.50 |
| | | | | | c=67.30 |
| 1.00 | 1.8 | 4.7 | 98.5 | 57493 | a=67.23 |
| | | | | | b=136.13 |
| | | | | | c=67.65 |
| 1.27 | 2.0 | 5.8 | 86.5 | 36870 | a=67.13 |
| | | | | | b=136.00 |
| | | | | | c=67.54 |
| R-merge indicates an error between each of a plurality of frames measured. | | | | | |

**Table 3**

| Results of Processing Diffraction Data of Native Crystal of Decarbamylase | | | | | |
|---|---|---|---|---|---|
| Wavelength | Resolution | R-merge | Yield | Independent reflection constant | lattice constant |
| (Å) | (Å) | (%) | (%) | | (Å) |
| 0.93 | 1.7 | 6.4 | 96.1 | 68596 | a=67.84 |
| | | | | | b=137.83 |
| | | | | | c=68.39 |
| R-merge indicates an error between each of a plurality of frames measured. | | | | | |

### (Example 5) Determination of Heavy Atomic Coordinates and Improvement in Phases

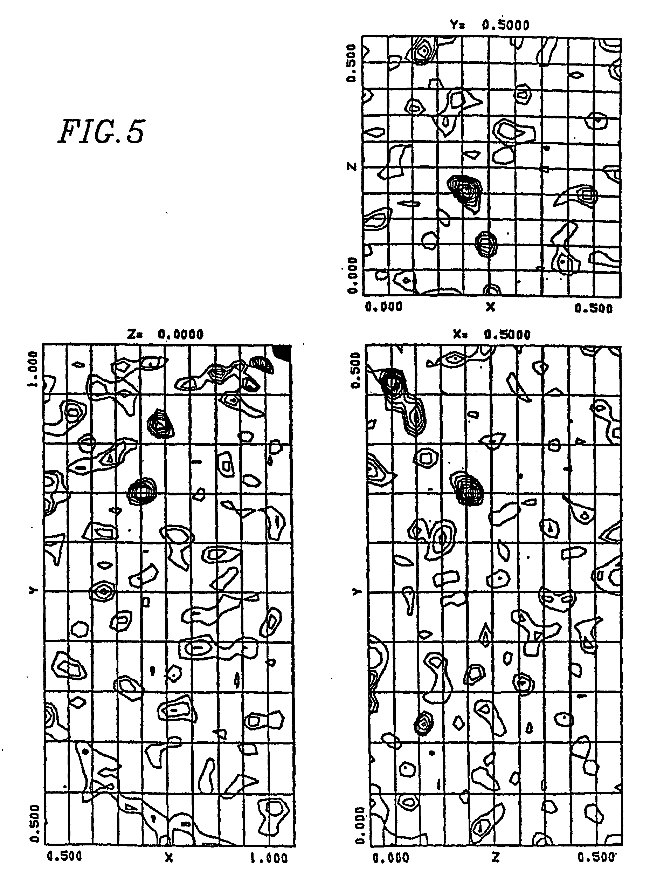

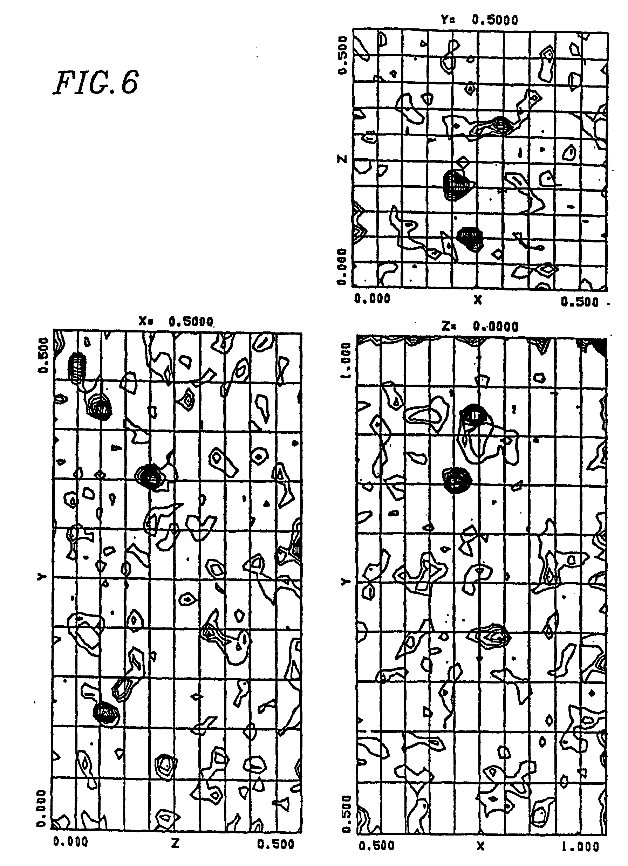

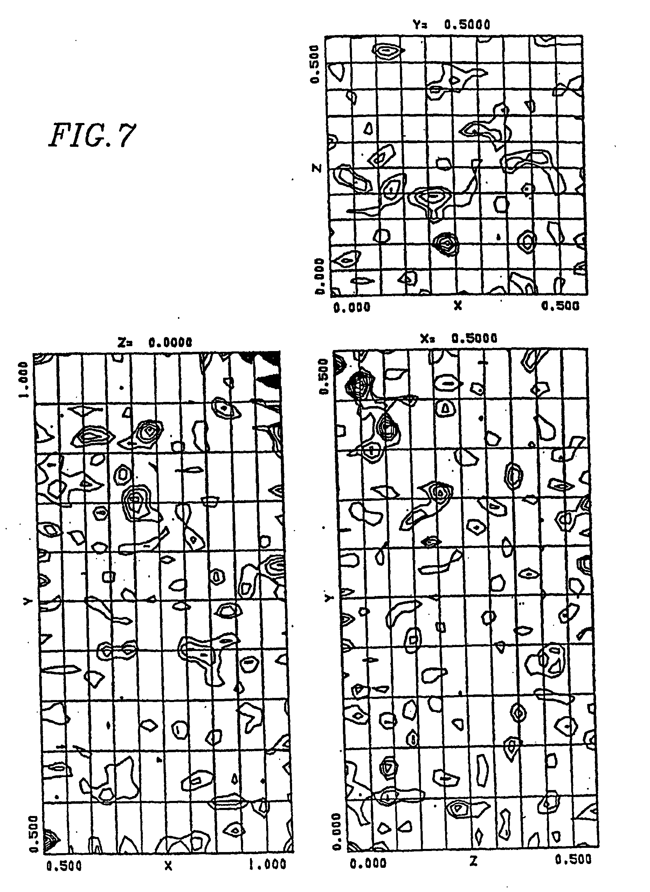

Out of the three wavelength diffraction data obtained for the EMTS derivative crystal, the data for 1.00 Å was assumed to be the data for the native crystal, the data for 0.98 Å was anomalous data, and the data for 1.27 Å was isomorphous data. The difference Patterson function and the anomalous difference Patterson function were calculated and the Harker plane (called a difference Patterson map, Figures **5** to **7**) was drawn. An attempt was made to identify the positions of heavy atoms from high intensity peak positions on the Harker plane and significant peak positions corresponding to the cross vectors. Patterson peaks having high intensity on each the Harker plane were selected from the two difference Patterson map (between the data of the 1.00-0.98 Å and between the data of 1.00-1.27 Å), and two mercury atoms (mercury 1 and mercury 2) were identified, and the coordinates thereof were calculated. To confirm this, a phase was first calculated using only the coordinate of mercury 1, and this phase was then used to calculate the coordinate of mercury 2 by difference Fourier calculation using the phase. Whether or not a peak from mercury 2 and a cross peak of mercury 1-mercury 2 were present in the difference Patterson map was then determined. In order to find the positions of other bound mercury atoms, heavy atom position parameters were refined and phase calculations were performed using the coordinates of mercury 1 and 2. The positions of the mercury atoms were calculated by difference Fourier calculation. Self peak and cross peaks were confirmed in a manner similar to when mercury 2 was identified. As a result, self and cross peaks corresponding to a further four mercury atoms could be confirmed, whereby the coordinates of mercury 3, mercury 4, mercury 5, and mercury 6 were determined.

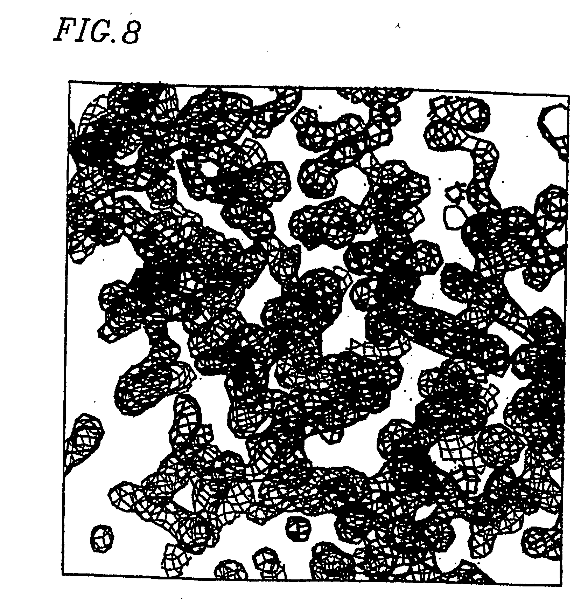

With 1.8 Å resolution data of the EMTS derivative crystal measured by 1.00 Å wavelength, the six calculated mercury atomic coordinates (heavy atom parameters) were refined using a program MLPHARE (CCP4 package)(SERC) to determine an initial phase. The average value of figure of merit after the refinement was 0.50. Thereafter, by performing a solvent flattening method and a histogram matching method with the program DM (CCP4 package, SERC), the phase was gradually extended from a low resolution to a high resolution (where the solvent region of the decarbamylase crystal was 35%), thereby improving the initial phase. It had been known from the measurement of crystal density that the decarbamylase crystal contains two molecules in a non-symmetrical unit. By utilizing the non-crystallographic symmetry, the electron density (called NCS averaging) was averaged to improve the phase. To apply this method, firstly, the noncrystallographic two-fold axis in the decarbamylase crystal was determined. With the phase after solvent flattening, the electron density map was calculated, and the positions of the heavy atoms and the electron density map were displayed on the three-dimensional graphics. It was found that two lines connecting between the middle points between the coordinates of the mercury atoms are perpendicular to each other on the Z axis (a crystallographic two-fold axis). Therefore, it was inferred that the decarbamylase molecule has three pieces of symmetry, i.e., two noncrystallographic two-fold axes and one crystallographic two-fold axis (222). Each section of the electron density map was observed in detail to determine the coordinates of the centers of the two molecules and calculate a non-crystallographic symmetry matrix including translation and rotation. At the same time, a region in which a protein molecule called a mask is present was identified from the electron density map obtained with the solvent flattening method. Based on the calculated non-crystallographic symmetry matrix and and the mask, NCS averaging calculation was performed by the program DM. As a result, a correlation coefficient between the two molecules was 0.92, indicating a high level of correlation, and the free R-factor was 24.5%. The improved electron density map was extremely clear (e.g., see Figure **8**; in Figure **8**, the linkage of a peptide principal chain was observed as a continuous electron density; in the middle of the figure, electron densities corresponding to the amino acid side chains identified as Glu46, Cys171, and Glu145 were observed). In secondary structure portions such as a helices or β sheets, the formation of amino acid principal chains could be clearly tracked, and the electron density of an aromatic amino acid side chain and the like were also clear.

### (Example 6) Construction and Refinement of Stereostructure of Decarbamylase Model

Based on the electron density map obtained in Example 5, the stereostructure of the decarbamylase model was assembled on the three-dimensional graphics with Program O (Uppsala Universitet). In order to match the electron density to the amino acid sequence, firstly, partial amino acid sequences having characteristic electron densities, such as a tryptophan residue, were found and the partial structure of amino acid residues matching the electron densities was constructed with reference to the amino acid sequence using Program O. By successively repeating this process, all of the amino acid residues of decarbamylase (303 residues) were matched to the corresponding electron densities, thereby constructing the initial stereostructure model of the entire molecule. The obtained stereostructure model was used as a starting model structure to refine the stereostructure in accordance with the refinement protocol of the stereostructure refining program XPLOR (XPLOR manual, Yale University). In this refinement process, a local structure which was largely shifted from the electron density map was corrected and the electron density corresponding to a water molecule was identified. The manipulation of including a water molecule was repeated in the refinement calculation while taking into consideration an R value and a free R-factor, resulting in further refinement. The R value of the final model structure of the native crystal including the water molecule was 19.6% for the reflection data of 500-1.7 Å resolution.

### (Example 7) Stabilization Design of Decarbamylase

An example of stabilization design of decarbamylase is provided, in which when an amino acid residue which is disadvantageous in terms of energy was mutated, an improvement in stability was observed. the dihedral angle of the principal chain (φ -60**°,** 4, ψ-44°) of proline at position 203 (Pro203) calculated from the stereostructure of the present invention has α dihedral angle which is characteristic to the a helix structure. In general, it is known that a proline residue serves as a residue which unstabilizes or destroys the helix structure. It is inferred that the presence of proline at position 203 in decarbamylase causes distortion in the principal chain structure to unstabilize the structure. Since this portion has a dihedral angle which is characteristic to the helix structure, by substituting the proline with an amino acid residue suitable for the helix structure, the naturally-occurring structural energy can be stabilized. For example, the proline may be substituted with an amino acid residue which contributes to formation of the α helix structure, such as alanine (Ala), glutamic acid (Glu), leucine (Leu), and serine (Ser). The amino acid at position 203 was mutated with twenty naturally-occurring amino acids mutation, and the structural energy of each structure was calculated with AMBER potential parameter (Weiner, P.A. et al., J. Comp. Chemistry 7(2) :230-252, 1986) to obtain an optimum amino acid mutation for this portion where the more the desired amino acid mutation, the lower the structural energy value. As a result, aspartic acid > threonine > serine > valine > glutamic acid > isoleucine > alanine > phenylalanine > tyrosine > leucine > histidine > asparagine > glutamine > cysteine > proline. The denaturating temperature of the amino acid mutants prepared in Example 1 is shown in Table 4 (International Publication No. WO94/03613). An improvement in stability (ΔTm=3.4-8.2°C) is recognized in any of the following mutations. It is reasonably considered that these improvements are obtained, since the distortion of the principal chain structure is removed by substituting proline with other amino acids. When proline was substituted with glutamic acid (ΔTm=8.2°C), the highest stability was obtained. This is because the mutation from proline to glutamic acid allows the carboxyl group of the side chain of the glutamic acid to form an ionic or hydrogen bond with the guanidino group of the side chain of arginine at position 139 around the carboxyl group. It is considered that such a newly added interaction contributes to an improvement in stability and, as a result, the stability higher than that of other mutants can be obtained. The reason why an increase in the stability of a His mutant is ΔTm=3.4°C which is smaller than that of other mutants is that when histidine is positively charged at pH 7.0, contribution to the stability by removal of the distortion of the principal chain is not present due to electrostatic repulsion between histidine and arginine at position 139.

The ordinal ranks of the stability of the optimum amino acid mutations at position 203 are not completely in agreement with the magnitudes of increases in the denaturating temperature experimentally obtained. Nevertheless, any of the mutations is positioned at a higher ordinal rank when counted from the mutations having lower structural energy (i.e., being more stable) than that of the proline residue of naturally-occurring decarbamylase, which is well in agreement with the experimental results.

**Table 4**

| Changes in Thermostability due to Amino Acid Mutations at Position 203 | | | | |
|---|---|---|---|---|
| Mutant strain | Mutation site | Amino acid mutation | Denaturaring temperature (°C) | ΔTm |
| 404 | Pro203 | Leu | 68.0 | 6.2 |
| 406 | Pro203 | Ser | 66.5 | 4.7 |
| 429 | Pro203 | Glu | 70.0 | 8.2 |
| 445 | Pro203 | Tbr | 67.5 | 5.7 |
| 468 | Pro203 | Ala | 67.7 | 5.9 |
| 469 | Pro203 | Ile | 67.2 | 5.4 |
| 470 | Pro203 | His | 65.2 | 3.4 |

Denaturating temperature is defined as temperature at which remnant specific activity is 50% where the above-described prepared (raw) enzyme solution is subjected to heat treatment for 10 minutes at each temperature followed by removal of insoluble matter due to thermal denaturation and the activity is thereafter measured. The remnant specific activity was measured as follows. N-carbamoyl-D-α-parahydroxyphenylglycine was dissolved in 0.1 M phosphate buffer (pH 7.0) to be 1.0 wt% substrate solution. 0.1 ml of enzyme solution was added to 1 ml of the substrate solution, followed by a reaction at 40°C for 20 minutes. The reaction was arrested by addition of 0.25 ml of trichloroacetic acid. The remnant specific activity was measured by subjecting the reaction solution to high performance liquid chromatography using D-phenylalanine where D-α-parahydroxyphenylglycine whose activity was transformed was used as internal standard. The mutation strains represented by identification numbers were obtained by screening. ΔTm indicates the difference in denaturating temperature between before and after mutation.

### INDUSTRIAL APPLICABILITY

The present invention provides decarbamylase, the stereostructure of a decarbamylase mutant, and the stereostructure model of the mutant. The stereostructure of the enzyme is useful for reasonable design of amino acid mutations aiming at stability in heat resistance, organic solvent resistance, air oxidation resistance, and the like; a change in the optimum pH of an enzyme reaction; and an improvement in specific activity. This design makes it possible to obtain an industrially useful modified enzyme in a speedy and efficient manner. Further, the stereostructure of the enzyme is the only one that has been determined among amidase or nitrilase which have stereostructure similar to that of the enzyme. It is apparent that the stereostructure of the enzyme can be really applied in the industrially applicable fields of these enzymes.

## Claims

1. Decarbamylase crystal having a space group P2₁2₁2 in the orthorhombic system and an amino acid sequence set forth in SEQ ID NO.: 1, or a space group P2₁2₁2₁ in the orthorhombic system and an amino acid sequence set forth in SEQ ID NO.: 2.

2. Decarbamylase crystal according to claim 1, wherein the crystal has a unit cell in the form of a rectangular parallelepiped and has lattice constants: a=66.5-68.5 Å, b=135.5-138.0 Å, and c=66.5-68.5 Å; and the amino acid sequence is SEQ ID NO.: 1.

3. Decarbamylase crystal according to claim 1, wherein the crystal has a unit cell in the form of a rectangular parallelepiped and has lattice constants: a=68.5-70.5 Å, b=138.0-140.5 Å, and c=68.5-73.0 Å; and the amino acid sequence is SEQ ID NO.: 1.

4. Decarbamylase crystal according to claim 1, wherein the crystal has a unit cell in the form of a rectangular parallelepiped and has lattice constants: a=81.5-82.5 Å, b=133.0-135.0 Å, and c=119.5-121.5 Å; and the amino acid sequence is SEQ ID NO.: 2.

5. Crystal according to any one of claims 1-4, wherein the crystal contains at least one or more heavy metal atoms per decarbamylase molecule.

6. Crystal according to claim 5, wherein the heavy metal atom is any of mercury, gold, platinum, lead, iridium, osmium, and uranium.

7. Frozen crystal, prepared by freezing decarbamylase crystal according to any one of claims 1-6 in liquid nitrogen.

8. A method for preparing a crystal of decarbamylase, comprising the steps of: providing decarbamylase solution having a concentration of 1-50 mg/ml; providing precipitant solution containing polyethylene glycol (PEG) or methoxypolyethylene glycol (PEGMME) having a concentration of 5-30 wt%, and a buffer agent having a concentration such that pH 6.0-9.0 is provided; mixing the decarbamylase solution with the precipitant solution; and allowing the resultant mixture solution to stand for a predetermined period of time until the decarbamylase crystal is grown in the solution to a predetermined size or more.

9. A method according to claim 8, wherein the mixing step comprises mixing a droplet of the decarbamylase solution with a droplet of the precipitant solution, and the step of allowing the resultant mixture solution to stand comprises suspending the mixture droplet obtained in the mixing step on a solution reservoir holding the precipitant solution in a sealed container, wherein the precipitant solution in the solution reservoir has a vapor pressure lower than a vapor pressure of the mixture droplet.

10. A method according to claim 8, wherein the mixing step comprises mixing a droplet of the decarbamylase solution with a droplet of the precipitant solution, and the step of allowing the resultant mixture solution to stand comprises suspending the mixture droplet obtained in the mixing step on a droplet stage of a solution reservoir holding the precipitant solution in a sealed container, wherein the precipitant solution in the solution reservoir has a vapor pressure lower than a vapor pressure of the mixture droplet.

11. A method according to claim 8, wherein the predetermined period of time during which the mixture solution is allowed to stand is one day to three weeks.

12. A method according to claim 8, further comprising placing the decarbamylase solution in a size exclusion semi-permeable membrane after the step of providing the decarbamylase solution, wherein the mixing step comprises diffusing the precipitant solution through the semi-permeable membrane into the decarbamylase solution.

13. A method according to claim 8, wherein the mixing step comprises gradually adding the precipitant solution to the decarbamylase solution, and the step of allowing the resultant mixture solution to stand comprises allowing the mixture solution to stand in a sealed container.

14. Decarbamylase **characterized by** a stereostructure having protein stereostructure topology represented in the following figure: 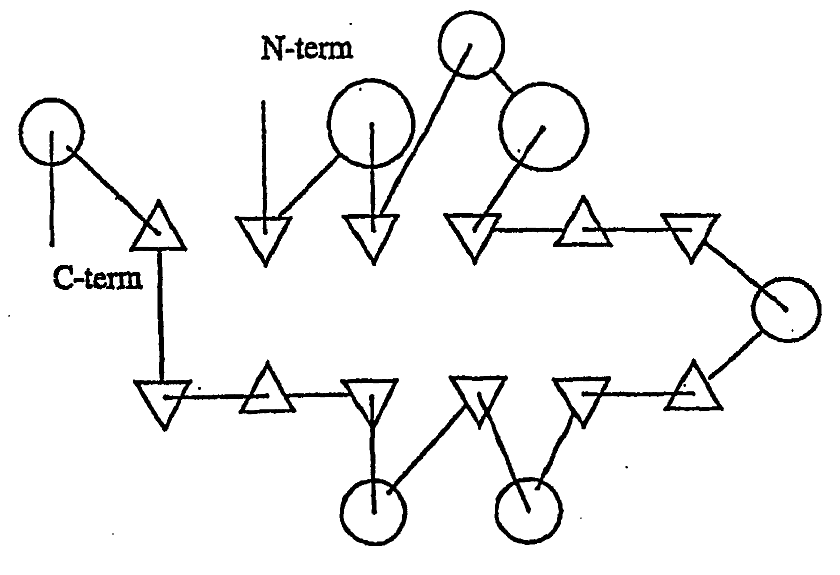 or an active fragment thereof.

15. Decarbamylase having a four-layer sandwich structure containing a secondary structure containing four α helices and twelve β sheets, or an active fragment thereof.

16. Decarbamylase, wherein amino acid residues thereof involved in an enzyme reaction are one cysteine residue, two glutamic acid residues; one lysine residue, and a substrate of the enzyme reaction is D-N-carbamoyl-α-amino acid; and a substrate-binding active site thereof is **characterized by** a stereostructure represented by: 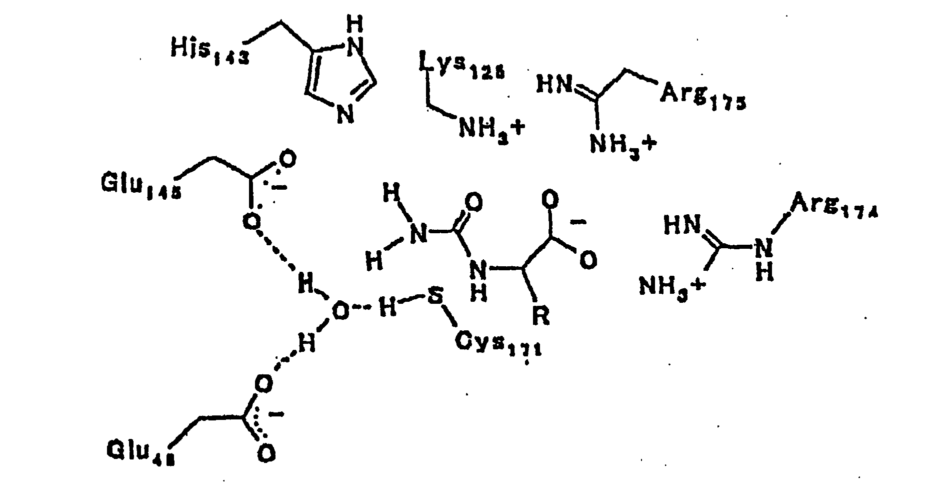 where a substitute R is a side chain of D-N-carbamoyl-α-amino acid, or a decarbamylase mutant, or an active fragment thereof.

17. An enzyme molecule having decarbamylase activity wherein a substrate thereof is D-N-carbamoyl-α-amino acid, wherein the enzyme molecule has an active site cavity formed of at least amino acids corresponding to the following amino acids of SEQ ID NO.: 1 or 2: Glu at position 46, Lys at position 126, Glu at position 145, and Cys at position 171, or an active fragment thereof.

18. An enzyme according to claim 17, wherein in a reaction, the D-N-carbamoyl-α-amino acid can interact with amino acids corresponding to Lys at position 126, His at position 143, Glu at position 145, Arg at position 174, Arg at position 175, and Thr at position 197 of SEQ ID NO.: 1 or 2 at the active site cavity, or an active fragment thereof.

19. An enzyme according to claim 17 or 18, wherein amino acids corresponding to Glu at position 46, Glu at position 145, and Cys at position 171 of SEQ ID NO.: 1 or 2 have a hydrogen bond via a water molecule at the active site cavity, or an active fragment thereof.

20. An enzyme molecule according to any one of claims 16-19, wherein the D-N-carbamoyl-α-amino acid is selected from the group consisting of D-N-carbamoyl-phenylglycine, D-N-carbamoyl-parahydroxyphenylglycine, D-N-carbamoyl-phenylalanine, D-N-carbamoyl-valine, D-N-carbamoyl-alanine, D-N-carbamoyl-cysteine, D-N-carbamoyl-aspartic acid, D-N-carbamoyl-glutamic acid, D-N-carbamoyl-glycine, D-N-carbamoyl-histidine, D-N-carbamoyl-isoleucine, D-N-carbamoyl-lysine, D-N-carbamoyl-leucine, D-N-carbamoyl-methionine, D-N-carbamoyl-asparagine, D-N-carbamoyl-proline, D-N-carbamoyl-glutamine, D-N-carbamoyl-arginine, D-N-carbamoyl-serine, D-N-carbamoyl-threonine, D-N-carbamoyl-tryptophan, and D-N-carbamoyl-tyrosine, or an active fragment thereof.

21. A decarbamylase complex **characterized by** a stereostructure of a complex of decarbamylase, a mutant thereof, or an active fragment thereof, and D-N-carbamoyl-α-amino acid or D-α-amino acid, wherein the complex is constructed with a molecular design technique from a stereostructure of decarbamylase according to claim 14 or 15.

22. A method for designing decarbamylase mutants, comprising the step of designing the decarbamylase mutants having a physical property and/or a function modified based on a stereostructure of decarbamylase according to any one of claims 14, 16, and 21.

23. A method for designing decarbamylase mutants, comprising the step of: preparing a crystal of an enzyme having decarbamylase activity; determining a stereostructure of the crystal by subjecting the crystal to X-ray crystallography; and designing the decarbamylase mutants having an improved physical property and/or function based on the determined stereostructure.

24. A method according to claim 23, wherein the stereostructure is a stereostructure of decarbamylase according to any one of claims 14, 16, and 21.

25. A method for designing decarbamylase mutants, comprising the step of: preparing a crystal of an enzyme having decarbamylase activity; determining a stereostructure of the crystal by subjecting the crystal to X-ray crystallography; designing the decarbamylase mutants having an improved physical property and/or function based on the determined stereostructure; and producing the decarbamylase mutants.

26. A method according to claim 25, wherein the stereostructure is a stereostructure of decarbamylase according to any one of claims 14, 16, and 21.

27. A method according to claim 26, wherein the step of designing the decarbamylase mutants is intended for a modification of one or more characteristics of the enzyme selected from the group consisting of a change in substrate specificity, a change in specific activity, an improvement in stability, optimization of optimum pH, and a change in water solubility.

28. A method according to claim 27, wherein the modification of the characteristics of the enzyme includes an improvement in stability.

29. A method according to claim 28, wherein the mutant designing for an improvement in stability includes a mutation including substitution of an amino acid residue which leads to a reduction in activity due to air oxidation.

30. A method according to claims 27, wherein the modification of the characteristics of the enzyme includes a change in specific activity and optimization of optimum pH.

31. A decarbamylase mutant obtained with a production method according to any one of claims 25-30.

32. A method for modifying a polypeptide or protein enzyme having a primary amino acid sequence similar to that of decarbamylase by utilizing a stereostructure of decarbamylase crystal according to any one of claims 1-7, or a stereostructure of decarbamylase according to any one of claims 14, 16, and 21.

33. A system for designing decarbamylase mutants using a computer, comprising:
means for determining a stereostructure of crystal of an enzyme having decarbamylase activity; and
means for designing the decarbamylase mutants having a physical property and/or a function improved based on the determined stereostructure.

34. A computer readable recording medium recording a program for executing a process for designing decarbamylase mutants, wherein the designing process comprises the steps of:
inputting data of crystal of an enzyme having decarbamylase activity determined by subjecting the crystal to X-ray crystallography; and
designing the decarbamylase mutants having a physical property and/or a function improved based on the determined stereostructure.

35. A recording medium recording data describing a stereostructure of a decarbamylase mutant obtained by a process comprising the steps of:
inputting data of crystal of an enzyme having decarbamylase activity determined by subjecting the crystal to X-ray crystallography; and
designing the decarbamylase mutant having a physical property and/or a function improved based on the determined stereostructure.
